# EUROPEAN PATENT APPLICATION

(11) **EP 4 410 983 A1**
(43) Date of publication of application: **07.08.2024**
(21) Application number: 22876946.9
(22) Date of filing: 30.09.2022
(51) Int. Cl.: C12N 15/70, C12N 9/22, C12P 7/52, G01N 21/31, G01N 30/02

(54) **ESCHERICHIA COLI HAVING 3-HYDROXYPROPIONATE PRODUCTION ABILITY, WITH 3-HYDROXYPROPIONATE PRODUCTION GENE INSERTED INTO SAME, AND USE THEREOF**

(30) Priority: 30.09.2021 KR 20210129844; 26.11.2021 KR 20210166073
(71) Applicant: LG Chem, Ltd., Yeongdeungpo-gu Seoul 07336 (KR)
(72) Inventor: LEE, Jungyeon, Daejeon 34122 (KR); SUNG, Min Kyung, Daejeon 34122 (KR); KIM, Jae Hyung, Daejeon 34122 (KR)
(74) Representative: Goddar, Heinz J.
(86) International application number: PCT/KR2022/014761
(87) International publication number: WO 2023/055174

(57) **Abstract**

The present specification pertains to a microorganism and/or Escherichia coli and a use thereof, wherein the microorganism and/or Escherichia coli has 3-hydroxypropionate (3-HP) production ability, with a foreign 3-HP production gene inserted into the genomic DNA thereof, and has a use of measuring the production ability of the inserted 3-HP production gene.

## Description

### [TECHNICAL FIELD]

### Cross-reference to related application(s)

The present application claims the benefit of priority based on Korean Patent Application No. 10-2021-0129844 filed on September 30, 2021 and Korean Patent Application No. 10-2021-0166073 filed on November 26, 2021, and the entire contents disclosed in the documents of the corresponding Korean patent applications are incorporated as a part of the present description.

It relates to a microorganism and/or Escherichia coli having 3-hydroxypropionic acid (3-hydroxypropionate, 3-HP) producing ability, in which a foreign 3-hydroxypropionic acid (3-hydroxypropionate, 3-HP) producing gene is inserted into the genomic DNA, and uses thereof, and a method for determining 3-HP producing ability of the 3-HP producing gene inserted into genomic DNA of the *Escherichia coli* (*E. coli*) bacterium, comprising culturing the microorganism and/or *E. coli* bacterium.

### [BACKGROUND]

3-Hydroxypropionic acid (3-hydroxypropionic acid, 3-HP) is an important synthetic intermediate used in various chemical processes, and is a platform compound which can be converted into a variety of chemicals including acrylic acid, methyl acrylate, acrylamide, 1,3-propanediol, ethyl 3-HP, malonic acid, propiolactone and acrylonitrile, and the like. In addition, it has been actively studied in academia and industry, since it was selected as Top 12 value-added bio-chemical by U.S. Department of Energy (DOE).

3-HP can be produced through a chemical process using ethylene cyanohydrin, beta-iodopropionic acid, beta-bromopropionic acid, beta-chloropropionic acid, beta-propiolactone, acrylic acid, and the like as an intermediate, and can be produced through a biological process from glycerol. However, most of these chemicals have problems that they are toxic and carcinogenic, and consume a large amount of energy under the condition of high temperature and pressure, and discharge pollutants in large quantities.

Production of 3-HP is largely made up of two methods which are a chemical method and a biological method, but in case of the chemical method, as the initial material is expensive and toxic substances are generated during the production process, it is pointed out that it is not eco-friendly, so an eco-friendly bio-process is in the spotlight.

For 3-HP biosynthesis using a microorganism, conventionally, studies of one-step fermentation methods in which 3-HP production and cell growth occur simultaneously have been mainly studied, but there was a problem that the yield and productivity of 3-HP decreased as the substrate for 3-HP production was also used for cell growth and by-products such as acetate or lactate are generated, so it was tried to be improved through a 2-step preparation method in which 3-HP production and cell growth are performed separately.

However, in case of *E. coli* bacterium having a plasmid vector comprising a gene set for producing 3-HP from glycerol, it could not produce 3-HP when the 2-step culturing was applied in which cell growth and 3-HP production are separated using a culture medium and a minimal medium in a flask, and due to this, the 2-step culturing could not be applied, so it was necessary to simultaneously add a carbon source for cell growth and a carbon source consumed for 3-HP conversion into the minimal medium and the growth and proceed growth and production at the same time, and therefore, the cell concentration was low, and thus, it was not suitable for evaluating performance of a recombinant gene.

In addition, when culturing *E. coli* bacterium having the plasmid vector comprising the gene set, antibiotic input was essential to keep the vector maintenance and productivity constant, and concerns about antibiotic input cost and environmental contamination were raised accordingly, and the 3-HP production of the *E. coli* bacterium having the plasmid vector did not increase in proportion to the copy number, so it was difficult to measure the 3-HP producing ability of the gene in the plasmid vector.

### [DISCLOSURE]

### [TECHNICAL PROBLEM]

Under the above circumstance, the present inventors have confirmed 3-HP production through a 2-step preparation method in which 3-HP production and cell growth are performed separately by inserting a foreign 3-hydroxypropionic acid (3-hydroxypropionate, 3-HP) producing gene into genomic DNA, and have confirmed that the 3-HP production is proportional to the copy number of the genomic DNA inserted gene, thereby completing the present invention.

One embodiment provides a microorganism (for example, *E. coli* bacterium) having 3-HP producing ability, in which a foreign 3-HP producing gene is inserted into genomic DNA with at least one copy number.

Another embodiment provides a composition for determining 3-HP producing ability of the 3-HP producing gene inserted into genomic DNA of the microorganism (for example, *E. coli* bacterium).

Other embodiment provides a method for determining 3-HP producing ability of the 3-HP producing gene inserted into genomic DNA of the microorganism (for example, *E. coli* bacterium), comprising culturing a microorganism (for example, *E. coli* bacterium) having 3-HP producing ability, in which a foreign 3-hydroxypropionic acid (3-hydroxypropionate, 3-HP) producing gene is inserted into genomic DNA with at least one copy number.

### [TECHNICAL SOLUTION]

The present description provides technologies related to a microorganism and/or *E. coli* bacterium having 3-HP producing ability, in which a foreign 3-hydroxypropionic acid (3-hydroxypropionate, 3-HP) producing gene is inserted into the genomic DNA, and uses thereof, and a method for determining 3-HP producing ability of the 3-HP producing gene inserted in genomic DNA of the *E. coli* bacterium comprising culturing the microorganism and/or *E. coli* bacterium.

In the present description, "3-hydroxypropionic acid (3-hydroxypropionate, 3-HP)" corresponds to carboxylic acid, particularly, β-carboxylic acid, and is an acidic viscous liquid, and may be produced by a microorganism (for example, *E. coli* bacterium).

In the present description, "microorganism" may comprise a single cell bacterium, and for example, it may be the genus of Escherichia including *E. coli* bacterium.

In the present description, *"E. coli* bacterium" is the genus of Escherichia, and it may be *E. coli* DH5a, *E. coli* JM101, *E. coli* K12, *E. coli* W3110, *E. coli* X1776, *E. coli* B, and/or *E. coli* XL1-Blue.

One embodiment provides a microorganism (for example, *E. coli* bacterium) having 3-HP producing ability, in which a foreign 3-hydroxypropionic acid (3-hydroxypropionate, 3-HP) producing gene is inserted into genomic DNA with one copy number or more.

Another embodiment provides a method for determining 3-HP producing ability of a 3-HP producing gene in which genomic DNA of the microorganism (for example, *E. coli* bacterium), comprising culturing a microorganism (for example, *E. coli* bacterium) having 3-HP producing ability, in which a foreign 3-hydroxypropionic acid (3-hydroxypropionate, 3-HP) producing gene is inserted into genomic DNA with one copy number or more.

Other embodiment provides a composition for 3-HP production, comprising a microorganism (for example, *E. coli* bacterium) having 3-HP producing ability, in which a foreign 3-hydroxypropionic acid (3-hydroxypropionate, 3-HP) producing gene is inserted into genomic DNA with one copy number or more.

Other embodiment provides a method for producing 3-HP, comprising culturing a microorganism (for example, *E. coli* bacterium) having 3-HP producing ability, in which a foreign 3-hydroxypropionic acid (3-hydroxypropionate, 3-HP) producing gene is inserted into genomic DNA with one copy number or more. The method for producing 3-HP may further comprise separating and/or purifying 3-HP form the culture, after the culturing.

Other embodiment provides a use for determining 3-HP producing ability of a 3-HP producing gene inserted into the microorganism (for example, *E. coli* bacterium) genomic DNA of the microorganism (for example, *E. coli* bacterium) having 3-HP producing ability, in which a foreign 3-hydroxypropionic acid (3-hydroxypropionate, 3-HP) producing gene is inserted into genomic DNA with one copy number or more.

Other embodiment provides a use for 3-HP production of a microorganism (for example, *E. coli* bacterium) having 3-HP producing ability, in which a foreign 3-hydroxypropionic acid (3-hydroxypropionate, 3-HP) producing gene is inserted into genomic DNA with one copy number or more.

The 3-HP producing gene may comprise a gene of at least one enzyme selected from the group consisting of glycerol dehydratase, aldehyde dehydrogenase, glycerol dehydratase reactivase and adenosyltransferase, but not limited thereto.

The microorganism (for example, *E. coli* bacterium) may be one in which a gene selected from the group consisting of yqhD, glpK, IdhA, ack-pta and gldA among endogenous genes is deleted, but not limited thereto.

In the culturing the microorganism (for example, *E. coli* bacterium), a gene selected from the group consisting of yqhD, glpK, IdhA, ack-pta and gldA among genes of the *E. coli* bacterium may be deleted, but not limited thereto.

The 3-HP producing gene may be inserted where at least one gene selected from the group consisting of IdhA, gldA, glpK, yqhD, mgsA, poxB, nfrA, fhuA, fadL, adhE, pflB and aldA genes in genome of the microorganism (for example, *E. coli* bacterium) is located, but not limited thereto.

The 3-HP producing gene may be inserted into genomic DNA by CRISPR-Cas9 system, but not limited thereto.

The microorganism (for example, *E. coli* bacterium) having 3-HP producing ability may have 3-HP producing ability increased in proportion to the copy number of the 3-HP producing gene, but not limited thereto.

Other embodiment provides a composition for determining 3-HP producing ability of the 3-HP producing gene inserted into genomic DNA of the microorganism (for example, *E. coli* bacterium), comprising the microorganism (For example, *E. coli* bacterium).

The determining 3-HP producing ability may be determining whether the 3-HP production produced by the *E. coli* bacterium is proportional to the copy number of the 3-HP producing gene inserted into genomic DNA of the *E. coli* bacterium, but not limited thereto.

The method for determining 3-HP producing ability of the 3-HP producing gene inserted into genomic DNA of the microorganism (for example, *E. coli* bacterium) may further comprise measuring the amount of 3-HP produced by the microorganism (For example, *E. coli* bacterium), but not limited thereto.

In the measuring the amount of 3-HP produced, by determining whether the 3-HP production produced by the *E. coli* bacterium is proportional to the copy number of the 3-HP producing gene inserted into genomic DNA of the *E. coli* bacterium, the 3-HP producing ability may be confirmed, but not limited thereto.

The method for determining 3-HP producing ability of the 3-HP producing gene inserted into genomic DNA of the microorganism (for example, *E. coli* bacterium) may not comprise administering an antibiotic, but not limited thereto.

The method for determining 3-HP producing ability of the 3-HP producing gene inserted into genomic DNA of the microorganism (for example, *E. coli* bacterium) may be characterized in that the 3-HP producing ability is increased in proportion to the copy number of the 3-HP producing gene, but not limited thereto.

In the method provided in the present description, the culturing the microorganism (for example, *E. coli* bacterium), may comprise,
the first culturing step which is culturing in a medium comprising glucose and/or sucrose as a carbon source; and
the second culturing step which is culturing in a medium comprising glycerol as a carbon source,
but not limited thereto.

The medium of the second culturing step may not comprise glucose and/or sucrose as a carbon source, but not limited thereto.

Hereinafter, the present application will be described in more detail.

### [DETAILED DESCRIPTION]

The microorganism according to one embodiment may have ability to synthesize an organic acid, and for example, it may have ability to synthesize 3-hydroxypropionic acid (or 3-HP producing ability). The microorganism according to one embodiment may be a strain genetically engineered so as to produce an organic acid, or to have ability to produce an organic acid or to intensify ability to produce an organic acid, by comprising a gene involved in organic acid production by itself. The organic acid production includes that produced in a strain, that is produced in a cell and secreted to outside of the cell, or a combination thereof.

In the present application, "having 3-HP producing ability" may mean a cell and/or a microorganism naturally having 3-HP producing ability, or a microorganism in which 3-HP producing ability is given to a parent strain having no 3-HP producing ability. It may be used to mean a case in which the microorganism has 3-HP producing ability by introducing a mutation according to one embodiment and/or a case in which the microorganism has 3-HP producing ability by introducing a mutation according to one embodiment into a microorganism having no 3-HP producing ability. For example, the microorganism of the genus Escherichia having 3-HP producing ability may mean a natural microorganism itself or a microorganism of the genus Escherichia having improved 3-HP producing ability by inserting an external gene related to 3-HP production mechanism or intensifying or inactivating an intrinsic gene.

In the present description, in the microorganism in which a foreign 3-hydroxypropionic acid (3-hydroxypropionate, 3-HP) producing gene is inserted into genomic DNA, insertion of the 3-HP producing gene into genomic DNA may be achieved by a composition and/or a system for multiple insertion of a target gene (for example, a foreign 3-hydroxypropionic acid (3-hydroxypropionate, 3-HP) producing gene) comprising the following, but not limited thereto:
(1) a recombinant vector, comprising a gene encoding Cas9 protein and a gene encoding recombinase ("common recombinant vector");
(2) a recombinant vector ("variable recombinant vector") set specific to a target site which is a site which is present on genome of a target cell and a target gene (for example, foreign 3-hydroxypropionic acid (3-hydroxypropionate, 3-HP) producing gene) is introduced into:
   i) a recombinant vector comprising a temperature sensitive origin (ts origin) and a gene expressing guide RNA (gRNA) specific to the target site ("G recombinant vector"); and
   ii) a recombinant vector comprising a target gene and a homologous region before and after the target site at both ends of the gene, in which the PAM sequence (NNGG) in the homologous region is substituted with CAAA ("D recombinant DNA").

The composition for multiple insertion may not comprise an antibiotic-resistant gene in the common recombinant vector, G recombinant vector and D recombinant DNA. By not comprising an antibiotic-resistant gene, there are advantages in that the size of each vector may be kept small, and the metabolic burden of a target cell may be reduced, and a target gene may be repeatedly inserted without limitation of the number of genes which can be introduced by the antibiotic-resistant gene without the antibiotic-resistant gene.

In the present description, "copy number" may mean the total number of the 3-HP producing gene or 3-HP producing gene cassette repeated equally in the microorganism (host cell; for example, *E. coli* bacterium), and the 3-HP producing gene or 3-HP producing gene cassette may be artificially introduced (inserted) from the outside. The 3-HP producing gene or 3-HP producing gene cassette may be inserted into genome of the microorganism as suggested in the present application, and/or present outside the genome through a structure such as a plasmid and the like. The 3-HP producing gene may mean a coding gene of at least one enzyme involved in a metabolic pathway accompanied for 3-HP production, and for example, it may be at least one selected from the group consisting of dhaB1,2,3, gdrA, gdrB, araE (aldH), btuR and the like or a combination thereof, but not limited thereto.

The 3-HP producing gene cassette comprises a series of gene combination related to 3-HP production, and may necessarily comprise the 3-HP producing gene described above and further comprise an expression (transcription) regulatory factor and/or a linker and the like. In one specific embodiment, the 3-HP producing gene cassette may comprise a 3-HP producing gene (for example, at least one selected from the group consisting of dhaB1,2,3, gdrA, gdrB, araE (aldH), btuR and the like or a combination thereof), an expression regulatory factor and a linker, and may be in a size of about 7kb, but not limited thereto, and for example, "the 3-HP producing gene was inserted into genomic DNA with 5 copy numbers or more" may mean that the above-mentioned 3-HP producing gene or 3-HP producing gene cassette of a total of 5 or more is inserted (repeated) into genome of a microorganism (host cell).

In the present description, "target cell" means a cell to be transformed, and the type may be selected without limitation within the range of the purpose of transformation. In one embodiment, the target cell may be a prokaryotic cell, and in another embodiment, the target cell may be a bacterial cell. In one embodiment, the target cell may include both gram negative groups and gram-positive groups, and in one specific embodiment, the target cell may be *E. coli* bacterium, but not limited thereto.

In the present description, "target gene" means a nucleic acid molecule to be inserted into genome of a target cell, and for example, it may be a foreign 3-hydroxypropionic acid (3-hydroxypropionate, 3-HP) producing gene.

In the present description, unless otherwise mentioned, the temperature means a Celsius temperature.

In the present description, unless otherwise mentioned, the base sequence was described from the 5' end to the 3' end, and the amino acid sequence was described from the N-terminus to the C-terminus.

In the present description, the composition and/or system for multiple insertion of a target gene comprises a common recombinant vector, comprising (1) a gene encoding Cas9 protein and a gene encoding recombinase.

The common recombinant vector does not require additional insertion after being first inserted into the target cell, so it can reduce burden on cells.

The common recombinant vector may comprise an operably linked inducible promoter, a gene encoding Cas9 protein and a gene encoding recombinase.

The inducible promoter may be used as being selected without limitation within the range of the purpose to regulate expression of the gene encoding Cas9 and/or gene encoding recombinase. In one example, the inducible promoter may be a promoter which is induced by arabinose.

The Cas9 protein performs a role of breaking (for example, double strand break (DSB)) by recognizing PAM (Protospacer adjacent Motif) among genome of the target cell. The PAM sequence may be NNGG sequence, and the "N" may be a "A", "T", "C" or "G" base.

The Cas9 protein may be derived from Streptococcus pyonenes, and may be encoded by the nucleic acid sequence of SEQ ID NO: 1, but not limited thereto.

When the double strand break produced by the Cas9 protein is not recovered by recombinase enzyme, and the like, cells die and are eliminated from the selection process, so it is possible to select cells without an antibiotic-resistant gene.

The recombinase may be selected without limitation within the range of the purpose for inserting a target gene into genome of a target cell based on a homologous region to be described later, and for example, it may be RED recombinase. In one embodiment, the recombinase enzyme may be encoded by the nucleic acid sequence of SEQ ID NO: 2, and when the target gene is inserted into genome in the target cell by the recombinase enzyme and therefore DSB is recovered, the death of the cell does not occur.

In the present description, the composition and/or system for multiple insertion of a target gene comprises (2) a variable recombinant vector set. The variable recombinant vector set comprises (i) a G recombinant vector (gRNA-containing) comprising a temperature sensitive origin and a gene expressing guide RNA (gRNA) specific to a target site, and (ii) a D recombinant DNA (Donor) comprising a target gene and a homologous region.

In the variable recombinant vector set, the gRNA and homologous region may be designed specifically to the target site according to the location of the target site of the target cell, and the design may be performed using a method known in the art without limitation.

The variable recombinant vector set comprises (i) a G recombinant vector comprising a temperature sensitive origin and a gene expressing guide RNA specific to a target site.

By culturing a transduced transformant at a certain temperature or more using the temperature sensitive origin, the G recombinant vector in the transformant may be removed.

In one embodiment, the certain temperature may be appropriately selected according to the type of the temperature sensitive origin, and for example, it may be 36 degrees Celsius or more, 37 degrees Celsius or more, 39 degrees Celsius or more, or 41 degrees Celsius or more, but not limited thereto.

It is possible to simply remove the G recombinant vector only by changing the G temperature, and it is possible to introduce a target gene by introducing a new G recombinant vector and a D recombinant vector specific to a new position, and thus multiple gene introduction is possible with a simple process.

The gene expressing guide RNA (gRNA) may be designed by appropriately selecting according to a target position at which a target gene is introduced by a person skilled in the art.

In one embodiment, the target gene of guide RNA may be at least one selected from the group consisting of IdhA, yqhD, glpK, gldA, mgsA, poxB, nfrA, fhuA, fadL, adhE, pflB and aldA genes among genes of a host cell (*E. coli* bacterium), but not limited thereto.

In one embodiment, the guide RNA may be at least one selected from the group consisting of the nucleic acid sequences of SEQ ID NOs: 3 to 17, but not limited thereto.

In addition, in the target cell to be transformed, a gene to be targeted by the guide RNA may be deleted, but not limited thereto.

When the gene to be targeted by the guide RNA is deleted in the target cell, the body part of the gene may be deleted, and the head and tail parts of the corresponding gene may be remained, and the guide RNA may act by targeting the genes of the head and tail parts.

The variable recombinant vector set comprises D (Donor) recombinant DNA, comprising (ii) a target gene and a homologous region.

The D recombinant DNA may be introduced in a linear DNA form into the target cell, and in one embodiment, the linear DNA may be prepared by cleaving a vector using restriction enzyme, or amplifying by polymerase chain reaction (PCR). In addition, the D recombinant DNA may be a recombinant vector or a DNA strand.

The homologous region corresponds to upstream and downstream pf a target site, and is attached to the 5' end and 3' end of the target gene, respectively. The homologous region may be appropriately adjusted in its length as needed, and for example, it may be 50 to 1200bp, 50 to 1000bp, 50 to 800bp, 50 to 700bp, 50 to 600bp, 50 to 500bp, 100 to 1200bp, 100 to 1000bp, 100 to 800bp, 100 to 700bp, 100 to 600bp, 100 to 500bp, 200 to 1200bp, 200 to 1000bp, 200 to 800bp, 200 to 700bp, 200 to 600bp, 200 to 500bp, 300 to 1200bp, 300 to 1000bp, 300 to 800bp, 300 to 600bp, or 300 to 500bp, respectively, but not limited thereto.

When the length of the homologous region is too short, the efficiency of homologous recombination may decrease, and when the homologous region is too long, the size of the vector to be introduced into a cell becomes too large, and therefore, the cell introduction efficiency may decrease.

When the target cell is *E. coli* bacterium, the homologous region preferably has a length of at least 50bp or more, and when the length of the homologous region is less than 50bp, the efficiency of homologous recombination may decrease. The length of the homologous region may be designed to be 100bp or more or 500bp or more in order to facilitate distinction of success experimentally.

The homologous region may be designed by appropriately selecting it according to a target position to which a target gene is to be introduced by a person skilled in the art, and in one embodiment, the homologous region may be the nucleic acid sequence of SEQ ID NOs: 18 to 31.

The homologous region (or homologous arm) attached to the 5' end of the target gene in the homologous region comprises a nucleic acid sequence in which the PAM (protospacer adjacent motif) sequence is substituted with other sequence. The PAM sequence is known to play a role of designating a location which Cas9 protein (Cas restriction enzyme) cleaves, and generally, it has the sequence of NGG. Then, each "N" base of the PAM sequence means a "A", "T", "G" or "C" base. In the present description, the PAM sequence may mean "NNGG", further comprising one base at the 5' end of the generally known "NGG" sequence.

The nucleic acid sequence substituting the PAM sequence may be used without limitation as long as it is not the sequence of 'NNGG' within the range of the purpose to prevent from being recognized specifically to Cas9, and for example, the NNGG nucleic acid sequence may be substituted with a nucleic acid sequence of NNAA, NNAT, NNAG, NNAC, NNTA, NNTT, NNTG, NNTC, NNCA, NNCT, NNCG, NNCC, NNGA, NNGT, or NNGC.

When the homologous region in which the PAM sequence is not substituted is attached to the 5' end of the target gene, there is a risk that Cas9 protein re-recognizes the corresponding site and cleaves it again while repeatedly introducing the target gene.

In the present description, the preparation method of a transformant comprises the following steps:
(1) introducing the composition and/or system provided by the present invention into a target cell;
(2) inserting a target gene to a target site of the target cell, by expressing Cas9 and recombinase in the target cell; and
(3) removing a G recombinant vector by culturing a cell in which the target gene is inserted into the target site at a temperature of 36 degrees Celsius or more.

The transformant prepared by the method may be one in which a target gene is inserted (multiple insertion or multiple introduction) into a plurality of target sites present on genome. The target gene inserted into each target site may be same and/or different from each other.

The preparation method of a transformant provided by the present invention comprises (1) introducing the composition and/or system provided by the present invention into a target cell.

Specific contents for the composition and/or system are as described above.

In the step (1), the common recombinant vector and variable recombinant vector set may be introduced simultaneously and/or sequentially, and they may be introduced by freely selecting a method known in the art within the range of the purpose of external vector introduction.

In the present description, the preparation method of a transformant comprises (2) inserting the target gene at the target site of the target cell, by expressing Cas9 and recombinase in the target cell.

The target site of the target cell is determined by gRNA expressed in the G recombinant vector introduced into the target cell, and Cas9 cleaves (for example, double strand break) the PAM sequence (NNGG) at the position designated by the corresponding gRNA.

The expressing Cas9 and/or recombinase in the target cell, may be performed by inducing using an inducible promoter. In one embodiment, the inducible promoter may be an inducible promoter by arabinose.

When the Cas9 and/or recombinase are always expressed, the metabolic burden of the target cell may increase, and a risk of damage to unintended genome site may increase.

In the present description, the preparation method of a transformant, comprises (3) removing a G recombinant vector by culturing a cell in which the target gene is inserted into the target site at a temperature of 36 degrees Celsius or more.

The removal of the G recombinant vector may be performed by the aforementioned temperature sensitive origin (ts origin). The culturing temperature may be appropriately selected according to the type of the temperature sensitive origin, and may be 36 degrees Celsius (hereinafter, same) or more, 37 degrees or more, 39 degrees or more or 41 degrees or more, but not limited thereto.

In the present description, the preparation method of a transformant, may further comprise the following steps, after the removing the G recombinant vector:
(i) introducing a variable recombinant vector set specific to a new target site other than a site which is present on genome of the transformant and the target gene is inserted into, into the transformant;
(ii) inserting the target gene into the new target site, by expressing Cas9 and recombinase in the transformant in which the variable recombinant vector set is introduced; and
(iii) removing a G recombinant vector by culturing the transformant in which the target gene is inserted into the new target site at a temperature of 36 degrees Celsius or more.

The step (i) to step (iii) may be repeated as many as the number of target sites present on genome of the target cell. The gRNA and/or homologous region in the variable recombinant vector set may be substituted and used for each target site.

There are advantages in that the target gene may be inserted into a plurality of target sites by the repeating step, and a large target gene with an overall length of 9kb or more or 10kb or more of the introduced target gene can be quickly and simply introduced into the genome of the target cell.

The present invention also provides a transformant prepared by the method.

The overall length of the target gene inserted in the transformant may be 1kb or more, 5kb or more, 10kb or more, 1 to 30kb, 1 to 15kb, 1 to 10kb, 5 to 30kb, 5 to 15kb, 5 to 10kb, 10 to 30kb, or 10 to 15kb, but not limited thereto.

The 3-HP producing gene of *E. coli* bacterium in which a foreign 3-hydroxypropionic acid (3-hydroxypropionate, 3-HP) producing gene is inserted into genomic DNA provided in the present description may be 1 copy number or more and 100 copy numbers or less, 2 copy numbers or more and 100 copy numbers or less, 3 copy numbers or more and 100 copy numbers or less, 4 copy numbers or more and 100 copy numbers or less, 5 copy numbers or more and 100 copy numbers or less, 6 copy numbers or more and 100 copy numbers or less, 7 copy numbers or more and 100 copy numbers or less, 10 copy numbers or more and 100 copy numbers or less, 12 copy numbers or more and 100 copy numbers or less, 1 copy number or more and 50 copy numbers or less, 2 copy numbers or more and 50 copy numbers or less, 3 copy numbers or more and 50 copy numbers or less, 4 copy numbers or more and 50 copy numbers or less, 5 copy numbers or more and 50 copy numbers or less, 6 copy numbers or more and 50 copy numbers or less, 7 copy numbers or more and 50 copy numbers or less, 10 copy numbers or more and 50 copy numbers or less, 12 copy numbers or more and 50 copy numbers or less, 1 copy number or more and 30 copy numbers or less, 2 copy numbers or more and 30 copy numbers or less, 3 copy numbers or more and 30 copy numbers or less, 4 copy numbers or more and 30 copy numbers or less, 5 copy numbers or more and 30 copy numbers or less, 6 copy numbers or more and 30 copy numbers or less, 7 copy numbers or more and 30 copy numbers or less, 10 copy numbers or more and 30 copy numbers or less, 12 copy numbers or more and 30 copy numbers or less, 1 copy number or more and 20 copy numbers or less, 2 copy numbers or more and 20 copy numbers or less, 3 copy numbers or more and 20 copy numbers or less, 4 copy numbers or more and 20 copy numbers or less, 5 copy numbers or more and 20 copy numbers or less, 6 copy numbers or more and 20 copy numbers or less, 7 copy numbers or more and 20 copy numbers or less, 10 copy numbers or more and 20 copy numbers or less, 12 copy numbers or more and 20 copy numbers or less, 1 copy number or more and 15 copy numbers or less, 2 copy numbers or more and 15 copy numbers or less, 3 copy numbers or more and 15 copy numbers or less, 4 copy numbers or more and 15 copy numbers or less, 5 copy numbers or more and 15 copy numbers or less, 6 copy numbers or more and 15 copy numbers or less, 7 copy numbers or more and 15 copy numbers or less, 10 copy numbers or more and 15 copy numbers or less, 12 copy numbers or more and 15 copy numbers or less, 1 copy number or more and 12 copy numbers or less, 2 copy numbers or more and 12 copy numbers or less, 3 copy numbers or more and 12 copy numbers or less, 4 copy numbers or more and 12 copy numbers or less, 5 copy numbers or more and 12 copy numbers or less, 6 copy numbers or more and 12 copy numbers or less, 7 copy numbers or more and 12 copy numbers or less, 10 copy numbers or more and 12 copy numbers or less, 1 copy number or more and 10 copy numbers or less, 2 copy numbers or more and 10 copy numbers or less, 3 copy numbers or more and 10 copy numbers or less, 4 copy numbers or more and 10 copy numbers or less, 5 copy numbers or more and 10 copy numbers or less, 6 copy numbers or more and 10 copy numbers or less, 7 copy numbers or more and 10 copy numbers or less, 1 copy number or more and 8 copy numbers or less, 2 copy numbers or more and 8 copy numbers or less, 3 copy numbers or more and 8 copy numbers or less, 4 copy numbers or more and 8 copy numbers or less, 5 copy numbers or more and 8 copy numbers or less, 6 copy numbers or more and 8 copy numbers or less, 7 copy numbers or more and 8 copy numbers or less, 1 copy number or more and 7 copy numbers or less, 2 copy numbers or more and 7 copy numbers or less, 3 copy numbers or more and 7 copy numbers or less, 4 copy numbers or more and 7 copy numbers or less, 5 copy numbers or more and 7 copy numbers or less, 6 copy numbers or more and 7 copy numbers or less, 1 copy number or more and 6 copy numbers or less, 2 copy numbers or more and 6 copy numbers or less, 3 copy numbers or more and 6 copy numbers or less, 4 copy numbers or more and 6 copy numbers or less or 5 copy numbers or more and 6 copy numbers or less, and for example, may be 5 copy numbers, 6 copy numbers, 7 copy numbers, 8 copy numbers, 9 copy numbers, 10 copy numbers, 11 copy numbers or 12 copy numbers, but not limited thereto.

In one embodiment, the microorganism (for example, *E. coli* bacterium) may be a strain which has 3-HP producing ability or is genetically engineered to have 3-HP producing ability. The production of 3-HP includes that produced in the strain, that produced in a cell and secreted outside the cell, or a combination thereof.

In the present description, "glycerol dehydratase" is enzyme that catalyzes a chemical reaction, and belongs to the family of lyase, in particular, hydrolase, that cleaves carbon-oxygen bonds, and participates in glycerol lipid metabolism, and uses a cofactor, cobalamin. The enzyme may be one encoded by dhaB (GenBank accession no. U30903.1) gene, but not limited thereto.

The dhaB gene is derived from Klebsiella pneumonia, and the gene encoding glycerol dehydratase may comprise genes encoding dhaB1, dhaB2 and/or dhaB3, but not limited thereto. The glycerol dehydratase protein and gene encoding this may comprise a mutation of the gene and/or amino acid sequence within the range of maintaining the enzymatic activity of degrading glycerol into -hydroxypropanal (3-HPA) and water (H₂O).

In the present description, "aldehyde dehydrogenase" is enzyme that catalyzes oxidation of aldehyde. The enzyme may convert aldehyde (R-C(=O)-H) into carboxylic acid (R-C(=O)-O-H). The enzyme may be encoded by aldH gene, and the gene may be aldH (GenBank Accession no. U00096.3; EaldH) gene derived from Escherichia coli or *E. coli* bacterium K12 MG1655 18-3 cell line, puuC gene derived from Klebsiella pneumonia (K. pneumonia) and/or KGSADH gene derived from Azospirillum brasilense, but not limited thereto. The aldehyde dehydrogenase protein and gene encoding this may comprise a mutation of the gene and/or amino acid sequence within the range of maintaining activity to produce 3-HP from 3-HPA.

In the present description, "glycerol dehydratase reactivase" acts to maintain the activity of the catalyst during the catalyst reaction of glycerol dehydratase. The enzyme may be encoded by gdrAB gene, and may be derived from Klebsiella pneumonia.

In the present description, "adenosyltransferase" plays a role of improving vitamin B₁₂ reuse, and may be encoded by gene btuR (GenBank: AAA23530.1), and this may be derived from Escherichia coli.

In the present description, "yqhD" is a gene encoding alcohol dehydrogenase, and encodes enzyme which converts 3-hydroxypropanal into 1,3-PDO when glycerol is degraded into 3-hydroxypropanal and water by glycerol dehydratase.

In the present description, "glpK" may be a gene encoding glycerol kinase.

In the present description, "IdhA" may be a gene encoding lactate dehydrogenase.

In the present description, "gldA" may be a gene encoding glycerol dehydrogenase.

In the present description, "ack-pta" may be a gene encoding acetate kinase-phosphate acetyltransferase.

In the present description, "mgsA" may be a gene encoding methylglyoxal synthase.

In the present description, "poxB" may be a gene encoding pyruvate dehydrogenase.

In the present description, "nfrA" may be a gene encoding bacteriophage adsorption protein A.

In the present description, "vector" refers to any vehicle for cloning and/or transition of a base into a host cell. The vector may be a replicon capable of bringing about replication of fragments bound by binding other DNA fragments. "Replicon" may mean any genetic unit (for example, plasmid, phage, cosmid, chromosome, virus), which functions as a self-unit of DNA replication in vivo, i.e. capable of replication by its own regulation. In the present invention, the vector is not particularly limited as long as it can replicate in a host, and any vector known in the art may be used. The vector used for construction of the recombinant vector may be a plasmid, cosmid, virus and/or bacteriophage in a natural state or recombinant state. For example, as the phage vector or cosmid vector, pWE15, M13, λEMBL3, λEMBL4, λHIXII, λDASHII, λZAPII, λgt10, λgt11, Charon4A, and/or Charon21A, and the like may be used, and as the plasmid vector, pDZ vector, pBR-based, pUC-based, pBluescriptll-based, pGEM-based, pTZ-based, pCL-based, and/or pET-based, and the like may be used. The usable vector is not particularly limited, and a known expression vector may be used. In one embodiment, the vector may be one such that a gene transferred by being inserted into the vector is irreversibly fused into genome of a host cell so that gene expression is stably maintained for a long time in the cell. This vector, may comprise a transcription and translation expression regulatory sequence which allows the corresponding gene can be expressed in the selected host. As the expression regulatory sequence, any operator sequence for regulating transcription and/or a sequence regulating termination of transcription and translation may be comprised. In one embodiment, an initiation codon and a stop codon may be generally considered as a part of a nucleic acid sequence encoding a target protein, and they should exhibit action in a subject when a genetic construct is administered and may be in frame with a coding sequence. In addition, in case of the replicable expression vector, it may comprise a replication origin. In addition, it may appropriately comprise an enhancer, an untranslated region at the 3' end of the targeted gene, a selection marker (for example, antibiotic-resistant marker) and/or a replicable unit and the like. The vector may be self-replicating or may be integrated into host genomic DNA.

According to one embodiment, each element in the vector should be operably linked, and the linkage of these element sequences may be performed at a convenient restriction enzyme site by ligation (linkage), and when such a site is not present, it may be performed using a synthetic oligonucleotide adaptor or linker according to a common method.

In the present description, "transduction" refers to a phenomenon in which bacterial DNA is transferred to other bacteria through a bacteriophage, and is one of methods of horizontal gene transfer (HGT). Such transducible bacteriophage is called "transduction particle" or "transduction phage", and a cell that has received DNA from other bacteria is called "transductant".

In the present description, "transformation" is to produce a gene into a host cell so that is can be expressed in a host cell, and when the transformed gene can be expressed in the host cell, anything inserted in chromosome of the host cell or positioned outside the chromosome may be included without limitation. In addition, the gene includes DNA and/or RNA encoding a target protein. As long as the gene can be introduced and expressed into a host microorganism, the introduced form is not limited. For example, the gene may be introduced into a host microorganism in the form of an expression cassette, which is a gene construct including all elements necessary for self-expression. The expression cassette may commonly comprise expression regulatory elements such as a promoter, a transcription termination signal, a ribosome binding site and/or a translation termination signal and the like, which are operably linked to the gene. The expression cassette may be in the form of an expression vector capable of self-replication. In addition, the gene may be introduced into a host cell in its own form and be operably linked to a sequence required for expression in the host cell.

In the present description, the method for transforming includes anything as long as it is a method of introducing a gene into a cell, and may be performed by selecting a suitable standard technique as known in the art. For example, electroporation, calcium phosphate (CaPO4) precipitation, calcium chloride (CaCl2) precipitation, microinjection, retroviral infection, polyethylene glycol (PEG) method, DEAE-dextran method, cationic liposome method and/or lithium acetate-DMSO method may be used, but not limited thereto.

In the method for determining 3-HP producing ability of the 3-HP producing gene inserted into genomic DNA of the microorganism (for example, *E. coli* bacterium), comprising culturing a microorganism (for example, *E. coli* bacterium), the culturing may comprise the first culturing step and/or the first culturing step and the second culturing step, but not limited thereto.

The culturing a microorganism (for example, *E. coli* bacterium),
may comprise
the first culturing step which is culturing in a medium comprising glucose and/or sucrose as a carbon source; and
the second culturing step which is culturing in a medium comprising glycerol as a carbon source,
but not limited thereto.

The medium comprising glucose and/or sucrose as a carbon source may be LB medium, and the medium comprising glycerol as a carbon source may be M9 medium, but not limited thereto.

The culturing may be made according to an appropriate medium and culture conditions known in the art, and it should meet requirements of a specific strain in an appropriate manner, and may be appropriately modified by a person skilled in the art.

The culture method may comprise for example, batch culture, continuous culture, fed-batch culture, or a combination thereof, but not limited thereto.

The fed-batch culture may be carried out using a 5L fermenter (Working volume 2L), and may be carried out by performing high-concentration cell culture, but not limited thereto.

According to one embodiment, the medium may comprise various carbon sources, nitrogen sources and trace element components, and the recombinant cell may be cultured in a common medium containing a proper carbon source, a nitrogen source, an amino acid, a vitamin and the like while adjusting temperature and/or pH and the like. The carbon source that may be used includes sugars and carbohydrates such as glucose, sucrose, lactose, fructose, maltose, starch and/or cellulose, oils and fats such as soybean oil, sunflower oil, castor oil and/or coconut oil and the like, fatty acids such as palmitic acid, stearic acid, and/or linolenic acid, alcohols such as glycerol and/or ethanol, and organic acid such as acetic acid. These materials may be used individually or as a mixture, but not limited thereto. As the nitrogen that can be used, peptone, yeast extract, meat juice, malt extract, corn steep liquor, soybean wheat and urea or an inorganic compound, for example, ammonium sulfate, ammonium chloride, ammonium phosphate, ammonium carbonate and ammonium nitrate may be comprised. The nitrogen source may also be used individually or as a mixture, but not limited thereto. The phosphorus source that can be used may comprise potassium dihydrogen phosphate or dipotassium hydrogen phosphate or a sodium-containing salt corresponding thereto, but not limited thereto. Furthermore, the medium may contain a metal salt such as magnesium sulfate or iron sulfate necessary for growth, but not limited thereto. In addition, an essential growth substance such as amino acids and vitamins may be comprised. Moreover, precursors suitable for the medium may be used. The medium or individual components may be added batchwise or continuously by an appropriate method to the culture solution during the culturing process, but not limited thereto.

According to one embodiment, by adding a compound such as ammonium hydroxide, potassium hydroxide, ammonia, phosphoric acid and sulfuric acid to microbial culture solution by an appropriate method during culturing, pH of the culture solution may be adjusted. In addition, bubble generation may be inhibited using an antifoaming agent such as fatty acid polyglycol ester during culturing. Additionally, in order to maintain the aerobic state of the culture solution, oxygen or oxygen-containing gas (e.g., air) may be injected into the culture solution. The temperature of the culture solution may be 20°C to 45°C, 25°C to 40°C or 30°C to 37°C, and for example, may be 37°C. The culture period may be continued until a useful substance (for example, 3-HP) is obtained in a desired yield, and for example, may be 3 to 60 hours, 3 to 48 hours, 3 to 36 hours, 3 to 28 hours, 6 to 60 hours, 6 to 48 hours, 6 to 36 hours, 6 to 28 hours, 12 to 60 hours, 12 to 48 hours, 12 to 36 hours, 12 to 28 hours, 20 to 60 hours, 20 to 48 hours, 20 to 36 hours or 20 to 28 hours, and for example, may be 24 hours, but not limited thereto.

The separating and/or recovering 3-HP in the cultured microorganism (or recombinant cell) and/or culture medium may be collecting a targeted substance (for example, 3-HP) form the medium, culture solution or microorganism using an appropriate method known in the art according to the culture method. For example, a method of centrifugation, filtration, extraction, spraying, drying, evaporation, precipitation, crystallization, electrophoresis, fractional dissolution (for example, ammonium sulfate precipitation) and/or chromatography (for example, ion exchange, affinity, hydrophobic, liquid and size exclusion) and the like may be used, but not limited thereto. The culture medium may mean a medium in which a microorganism (or recombinant cell) is cultured.

In one embodiment, the method for producing 3-HP may additionally comprise purifying 3-HP before, at the same time of, or after the separating and/or recovering.

### [ADVANTAGEOUS EFFECTS]

In the present description, the microorganism and/or *E. coli* bacterium in which a foreign 3-hydroxypropionic acid (3-hydroxypropionate, 3-HP) is inserted into genomic DNA has 3-HP producing ability, and a composition for determining 3-HP producing ability of the 3-HP producing gene inserted into the genomic DNA comprising the microorganism and/or *E. coli* bacterium is provided. In addition, a method for determining 3-HP producing ability of the 3-HP producing gene inserted into the microorganism and/or *E. coli* bacterium genomic DNA is provided.

### [BRIEF DESCRIPTION OF THE DRAWINGS]

FIG. 1 is a cleavage map of the common recombinant vector comprising Cas9 gene and recombinase.
FIG. 2 shows the homology arm region connected before and after the insertion site to the 5' and 3' ends of the target gene comprised in the D recombinant DNA.
FIG. 3 shows the cassette recombinant vector comprising 4 kinds of target genes (inserted genes).
FIG. 4 shows the 3-HP producing gene plasmid vector, pCDF_J23101_dhaB_gdrAB_J23100_aldH_btuR vector.
FIG. 5 shows the 3-HP producing pRSF plasmid vector based on the pRSFDuet-1 vector.

### [MODE FOR INVENTION]

Hereinafter, the present invention will be described in detail by examples. However, the following examples illustrates the present invention only, but the present invention is not limited by the following examples.

Unless otherwise mentioned in the present description, all temperatures are based on degrees Celsius, and nucleic acid sequences are described in the direction from the 5' end to the 3' end unless otherwise specified.

### Example 1. Plasmid design for strain recombination

### 1-1. Preparation of common recombinant vector (comprising Cas9 and recombinase)

In the present example, a common recombinant vector comprising Cas9 gene and recombinase was prepared.

More specifically, under araBAD promoter induced RSF origin, arabinose, Cas9 gene (SEQ ID NO: 1) and recombinase (RED recombinase; SEQ ID NO: 2) were inserted into an araC gene and RED recombinase gene expression vector that regulates transcription of Cas9, to prepare a common recombinant vector, and the specific information was shown in Table 1 below.

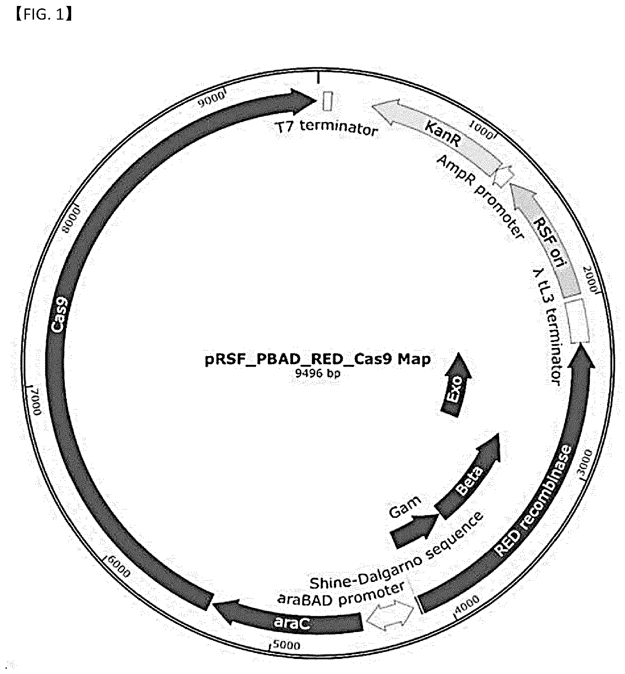

The cleavage map of the common recombinant vector was shown in FIG. 1. The common recombinant vector is always maintained in a recombinant cell without an additional gene insertion process after being inserted once at the time of the first gene insertion, and performs a function to edit genome according to the kind of gRNA to be introduced and homology region.

**[Table 1]**

| Name | Sequence (5'->3') | SEQ ID NO |
|---|---|---|
| Cas9 gene | tgagtcagctaggaggtgactga | 1 |
| RED recomgbinase gene | aaccatgcaggagattaacactctgctgatcgccctggataaaacatgggatgacgacttattgccgct ctgttcccagatatttcgccgcgacattcgtgcatcgtcagaactgacacaggccgaagcagtaaaagc tcttggattcctgaaacagaaagccgcagagcagaaggtggcagcatgacaccggacattatcctgca gcgtaccgggatcgatgtgagagctgtcgaacagggggatgatgcgtggc acaaattacggctcggcg tcatcaccgcttcagaagttcacaacgtgatagcaaaaccccgctccggaaagaagtggcctgacatg aaaatgtcctacttccacaccctgcttgctgaggtttgcaccggtgtggctccggaagttaacgctaaag cactggcctggggaaaacagtacgagaacgacgccagaaccctgtttgaattcacttccggcgtgaat gttactgaatccccgatcatctatcgcgacga aagtatgcgtaccgcctgctctcccgatggtttatgcag tgacggcaacggccttgaactgaaatgcccgtttacctcccgggatttcatgaagttccggctcggtggt ttcgaggccataaagtcagcttacatggcccaggtgcagtacagcatgtgggtgacgcgaaaaaatgc ctggtactttgccaactatgacccgcgtatgaagcgtgaaggcctgcattatgtcgtgattgagcgggat gaaaagtacatggcgagttttgacgagatcgtgccggagttcatcgaaaaaatggacgaggcactggc tgaaattggttttgtatttggggagcaatggcgatga | 2 |

### 1-2. G recombinant vector (comprising guide RNA and ts Origin)

Under temperature sensitive pSC101 origin, ampicillin antibiotic resistant gene and J23100 promoter, an expression vector comprising sgRNA designating an integration site and sgRNA scaffold (sequence that associates with Cas9) of CRISPR-Cas9 system of *Streptococcus pyrogenes* (*S. pyogenes*) was prepared, and named as G (Guide RNA-containing) recombinant vector.

The sgRNA may be prepared by a method known in the art depending on the insertion target site, and the nucleic acid sequence of the gene encoding the sgRNA used in the present invention was shown in Table 2 below.

**[Table 2]**

| Target | gRNA sequence (5'->3') | Accession Number | SEQ ID NO | Name of the sequence |
|---|---|---|---|---|
| IdhA | GCACGTTGTGGCAGGCAGAC | BAA14990.1 (GenBank) | 3 | IdhA_gRNA1 |
| | TAACGTCTGATTCAGAGAAC | | 4 | IdhA_gRNA2 |
| | AACGTCTGATTCAGAGAACA | | 5 | IdhA_gRNA3 |
| yqhD | ACTTTCCTGCTGGCGGTTGG | BAE77068.1 (GenBank) | 6 | yqhD_gRNA1 |
| | CACCAAATTTATCG CCG CAG | | 7 | yqhD_gRNA2 |
| glpK | ATTGTCTGGGAAAAAGAAAC | BAE77384.1(GenBank) | 8 | glpK_gRNA1 |
| | CGTCGTTCTTCCGAAGTATA | | 9 | glpK_gRNA2 |
| gldA | ATCGCGGAGACTGCGCAGTG | AAC76927.2 (GenBank) | 10 | gldA_gRNA1 |
| | CCTCGATACTGCCAAAGCAC | | 11 | gldA_gRNA2 |
| mgsA | CGAGATAACGCTGATAATCG | CAA72119.1 (GenBank) | 12 | mgsA_gRNA1 |
| | CGCAGCAAGGCTTTCACGTC | | 13 | mgsA_gRNA2 |
| poxB | CAGTGCATGGTTGCCCCTTC | CAA27725.1 (GenBank) | 14 | poxB_gRNA1 |
| | GTACTTGTGGGATCTGCTCC | | 15 | poxB_gRNA2 |
| nfrA | CGCATGCAGCCACCAGTAGA | AAC36849.1 (GenBank) | 16 | nfrA_gRNA1 |
| | CCAGTCGCGCCATTAAAGTC | | 17 | nfrA_gRNA2 |

### 1-3. D recombinant DNA (comprising homology region and target gene)

D (Donor) recombinant DNA comprising a target gene (donor or insert) to be introduced into genome was prepared as follows.

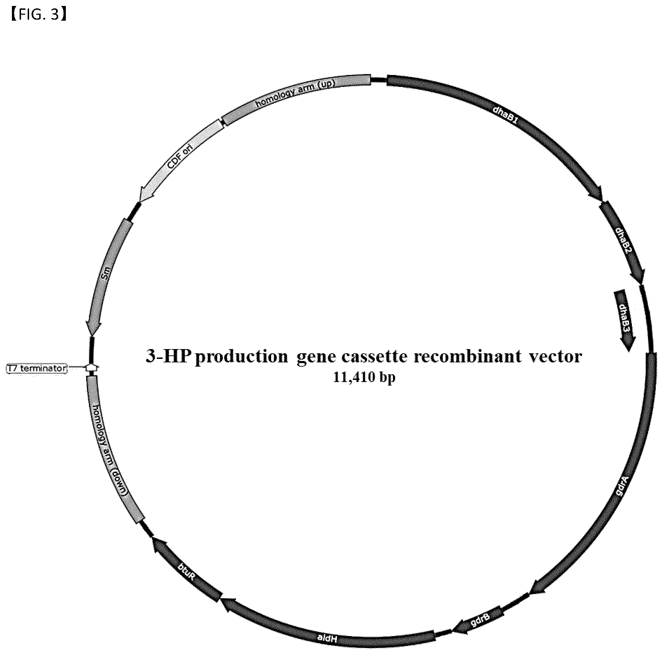

As the target gene, dhaB, gdrAB, aldH and btuR parts of the pCDF_J23101_dhaB_gdrAB_J23100_aldH_btuR vector of Korean Patent Publication No. 10-2020-0051375 were used, and homology arm regions with a length of 1kb were connected before and after the insertion site at the 5' and 3' ends of the target gene, respectively. Then, the PAM sequence (NNGG) comprised in the homology arm in the 5' direction (beginning part of foreign target gene operon) was substituted with CAAA to prepare a 3-HP producing gene cassette recombinant vector (See FIG. 3). In the PAM sequence, the N base means an A base, a T base, a C base or a G base.

Specifically, by treating xbal and pacl restriction enzymes (Thermo Scientific) to the pCDF_J23101_dhaB_gdrAB_J23100_aldH_btuR vector, fragments were prepared, and PCR was performed on the genomic DNA of the *E. coli* W3110 strain (purchased from KCRC) with primers of Table 3 below, and upstream and downstream fragments of the homology arm were prepared, and the two fragments were mixed by In-fusion (TAKARA) reaction to prepare a 3-HP producing gene cassette recombinant vector.

**[Table 3]**

| homology arm primer | homology arm sequence (5'->3') | SEQ ID NO |
|---|---|---|
| ldhA_upstream_For | actgaaccgctctaggcgattgggatgtgtgcattacc | 48 |
| ldhA_upstream_Rev | tagctgtaaatctagatctctctgcactgcccgc | 49 |
| ldhA_downstream_For | tactagcgcagcttaagctcaaagccaaaggactcg | 50 |
| ldhA_downstream_Rev | cagcagcctaggttaacgatcaattgcccgctattttcc | 51 |
| yqhD_upstream_For | actgaaccgctctagggcggagttaatcccgctg | 52 |
| yqhD_upstream_Rev | tagctgtaaatctagcactttctgttcgcgaaccagtttc | 53 |
| yqhD_downstream_For | tactagcgcagcttagaacagtatgttaccaaaccggttg | 54 |
| yqhD_downstream_Rev | cagcagcctaggttaagggctttgccgacacct | 55 |
| glpK_upstream_For | tactagcgcagcttaacctatggcactggctgct | 56 |
| glpK_upstream_Rev | tagctgtaaatctaggtcatattacagcgaagctttttgt | 57 |
| glpK_downstream_For | tactagcgcagcttaaagcgtttatgccgcatccg | 58 |
| glpK_downstream_Rev | cagcagcctaggttatcttcgttgtcgcctttgacg | 59 |
| gldA_upstream_For | actgaaccgctctagcatgcacgaagaattccgtaac | 60 |
| gldA_upstream_Rev | tagctgtaaatctagaaacctaaaacaaatttgtcaccc | 61 |
| gldA_downstream_For | tactagcgcagcttagaagcggcatgtgcagaagg | 62 |
| gldA_downstream_Rev | cagcagcctaggttatctgaaaccaccccaatatgtgc | 63 |
| mgsA_upstream_For | actgaaccgctctaggattgtgattctggagaaaggtcgc | 64 |
| mgsA_upstream_Rev | tagctgtaaatctagcggaccgtctgaagtaatattgc | 65 |
| mgsA_downstream_For | tactagcgcagcttatccgcgcaggtaaagtgc | 66 |
| mgsA_downstream_Rev | cagcagcctaggttacgaatgaatgagagcaaaagcggg | 67 |
| poxB_upstream_For | actgaaccgctctagcggcattaaagatgcgcgca | 68 |
| poxB_upstream_Rev | tagctgtaaatctagacaacagcgtgctgggct | 69 |
| poxB_downstream_For | tactagcgcagcttaagctcgcaatagtgactacattcgc | 70 |
| poxB_downstream_Rev | cagcagcctaggttacgtgatgacctgcggccc | 71 |
| nfrA_upstream_For | actgaaccgctctagtgtgcggccaggcgtcgtagtgc | 72 |
| nfrA_upstream_Rev | tagctgtaaatctaggggcttccggacgatccg | 73 |
| nfrA_downstream_For | tactagcgcagcttaggcgagcagtttttgcgc | 74 |
| nfrA_downstream_Rev | cagcagcctaggttaacagaaaaataacgacgaagcaacc | 75 |

By substituting the PAM sequence with the CAAA sequence, it is possible to prevent the foreign gene insertion site that has already been introduced and expressed in the subsequent repeated steps of transformation from being cleaved by Cas9 again.

In order to insert the 3-HP producing gene cassette recombinant vector, the sequences of the homology arms acting on the gene targeted to the guide RNA so that the guide RNA sequence of Table 2 acts on the *E. coli* genome to be inserted were shown in Table 4 below.

**[Table 4]**

| homology arm | homology arm sequence (5'->3') | SEQ ID NO |
|---|---|---|
| IdhA_upstream | ggcgattgggatgtgtgcattacccaacggcaaacgctgtagcgaacagtcacttgccgcc gggagctgtggcagctattaattcattaaatccgccagcttataagttaatgtctgttttgcgg tcgccagcgttaactggttcgcggtcagatccacttgtgcaccttctttcagcatttcgctaat ggtgttatcgagttcattaagctgcgggttagcgcacatcatacgggtcattgccagcccttt ggc | 18 |
| IdhA_downstream | tactagcgcagcttaagctcaaagccaaaggactcgttcacctgttgcaggtacttcttgtcg tactgttttgtgctataaacggcgagtttcataagactttctccagtgatgttgaatcacattt aagctactaaaaatattttacaaaatttcaaatttaattgaaagctatggcgatattgaaaa attcatcaacaactatgcttagtgtaggcgcaaccttcaactgaacggttaaacatgccaca atac | 19 |
| yqhD_upstream | ggcggagttaatcccgctggctgccatcgacggctggaaatgctcatcttcgccaatgtccat caacagttcctgtaactgcaaaatatcgacattgagacgcaaccctgccagcggcacctctg acgtggcataggtttcgcactcaaacggcaacggcaccgtcagcagcaggtattcattggc atcataacgaaacacgcgttcattgatataaccgattttatgcccggaaaagagaattatga tgccagg | 20 |
| yqhD_downstream | tactagcgcagcttagaacagtatgttaccaaaccggttgatgccaaaattcaggaccgttt cgcagaaggcattttgctgacgctaatcgaagatggtccgaaagccctgaaagagccaga aaactacgatgtgcgcgccaacgtcatgtgggcggcgactcaggcgctgaacggtttgattg gcgctggcgtaccgcaggactgggcaacgcatatgctgggccacgaactgactgcgatgca cggtctggat | 21 |
| glpK_upstream | ccgggttgttgattttcttcggtgtgggttgcgttgcagcactaaaagtcgctggtgcgtctttt ggtcagtgggaaatcagtgtcatttggggactgggggtggcaatggccatctacctgaccgc aggggtttccggcgcgcatcttaatcccgctgttaccattgcattgtggctgtttgcctgtttcg acaagcgcaaagttattccttttatcgtttcacaagttgccggcgctttctgtgctgcggctt | 22 |
| glpK_downstream | tactagcgcagcttaacctatggcactggctgctttatgctgatgaacactggcgagaaagc ggtgaaatcagaaaacggcctgctgaccaccatcgcctgcggcccgactggcgaagtgaa ctatgcgttggaaggtgcggtgtttatggcaggcgcatccattcagtggctgcgcgatgaaa tgaagttgattaacgacgcctacgattccgaatatttcgccaccaaagtgcaaaacaccaat ggtgtgtat | 23 |
| gldA_upstream | catgcacgaagaattccgtaacgcgctggttaacgccgcctctgccgacgctatcgccagcc tgctgcaacatgaactggaactgtaaaaggaaacatcatggaactgtatctggacaccgct aacgtcgcagaagtcgaacgtctggcacgcatattccccattgccggggtgacaactaacc cgagcattatcgctgccagcaaggagtccatatgggaagtgctgccgcgtctgcaaaaagc gattggtgat | 24 |
| gldA_downstream | tactagcgcagcttagaagcggcatgtgcagaaggtgaaaccattcacaacatgcctggcg gcgcgacgccagatcaggtttacgccgctctgctggtagccgaccagtacggtcagcgtttc ctgcaagagtgggaataacctactccaaactcccggcttgtccgggagtttgaacgcaaaat tgcctgatgcgctacgcttatcaggcctacgcaatctctgcaatatattgaatttgcgtgcttt tgtagg | 25 |
| mgsA_upstream | gattgtgattctggagaaaggtcgctttatcagcccgtggaagcatattctggttgatgaatt tcaggatatctcgccgcagcgggcagcgttgttagcggcattacgcaagcaaaacagtcag acgacgttgttcgctgttggtgatgactggcaggcgatttaccgattcagcggtgcgcaaat gtcgctcaccaccgctttccatgaaaactttggtgaaggcgaacgctgtgatttagacacga cttaccg | 26 |
| mgsA_downstream | tactagcgcagcttatccgcgcaggtaaagtgcgagtcgtcagttccataatgtacatccgt agttaactttcctacagattactgtaagcacttatcgctgcaagataaagaccgaaaaagcc tgcgcacaggcacaaaaatctcaggaagatggttgtttttccgcccactgcaggaaagtatt tcgcgtttgtgggtcagccagtttaaaccaatacttcagccgttgttctgtgagcacctgaga | 27 |
| | ctgcgg | |
| poxB_upstream | ttaccttagccagtttgttttcgccagttcgatcacttcatcaccgcgtccgctgatgattgcgc gcagcatatacaggctgaaacctttggcctgttcgagtttgatctgcggtggaatggctaact cttctttggcgaccaccacatccaccaacaccggaccgtcgatggagaaggcgcgttgcag ggcttcatcaacttcagacgctttttctacacggatacccgtaatgccgcacgcttcggcaat gcg | 28 |
| poxB_downstream | tactagcgcagcttaagctcgcaatagtgactacattcgcggaatagctcttgtgggtgggtt tcctggaaatagccgctgccaatttcgctggagggaatatgagcggcaatcgccagtaccg gaacgtgattgcggtggcaatcgaacaggccgttgattaagtgcaggttgccggggccgca cgatccggcgcagaccgccagttctccgctaagttgtgcttcagcgccagcggcaaaggcc gccacttct | 29 |
| nfrA_upstream | tgtgcggccaggcgtcgtagtgcgtctcgccggtccagatattccagcggaccccgactccg ccaagctgcgcgccctgagtgcctttatcacgatagccgttgtcctgaacgtgagcgtaagg ctcaatagtctgtccgttagctaccttctgatgccagctgacgcgataatctgccgtccacgc ctgaatatcctggcggatatattgcgccgcatcgaggtacaggttttgggcaaaccagcctg aaccgt | 30 |
| nfrA_downstream | tactagcgcagcttaggcgagcagtttttgcgctgcgtcgtactgaccttcttttaacagcacc ggtagcgtcgcgccaacaacatactggcggttgtcggcaaactgtaccgtataattcgccaa cgcctgaacggggttagcgctgtatttagataacagatagagccaacttttctcttgtgcgtc cgtggtaaatagtggcttattttcaatgagataatgctggaggcgtgctttttcgccacgata agc | 31 |

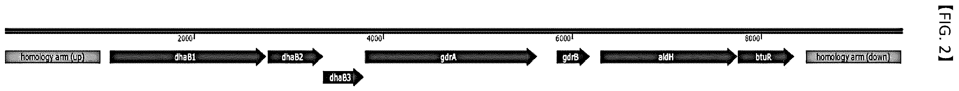

The homology arm connection part and the cassette recombinant vector were shown in FIG. 2 and FIG. 3, respectively.

### Example 2. Gene deletion and preparation of strain in which 3-hydroxypropionic acid (3-HP) producing gene is inserted into genomic DNA

The common recombinant vector, G recombinant vector and D recombinant DNA prepared in Example 1 were transduced into W3110 which was a strain in which *yqhD, glpK, IdhA, ack-pta* and *gldA* genes were deleted from *E. coli* W3110 strain (purchased from KCTC) (ΔyqhD, ΔgIpK, ΔIdhA, Δack-pta and LgIdA) (named as DKALG strain) with 5 copy numbers, 6 copy numbers and 7 copy numbers, respectively. After that, a transformant was prepared by expressing Cas9 and RED recombinase of the common recombinant vector.

Transducing 5 copy numbers into the DKALG strain was transducing using IdhA, gldA, glpK, yqhD and mgsA (5) sgRNA of Example 1-2 above as the G recombinant vector, and transducing 6 copy numbers was transducing using IdhA, gldA, glpK, yqhD, mgsA and poxB (6) sgRNA of Example 1-2 above, and transducing 7 copy numbers was transducing using IdhA, gldA, glpK, yqhD, mgsA, poxB and nfrA (7) sgRNA of Example 1-2 above.

Specifically, the *E. coli* strain was cultured to be between O.D (600nm) 0.5 to 1 under the condition of 37°C Celsius, and was washed with DW (distilled water) twice and then each recombinant vector 50 to 100ng was added for electroporation to select a colony. In case of the D recombinant DNA, it was introduced in a linear form or introduced as the vector as it is, by enzymatic treatment or performing PCR for the prepared vector.

Then, the selected colony was cultured in an LB medium at 37°C Celsius, and arabinose was added at a concentration of 0.1 to 1%(w/v) to express RED recombinase, and was further cultured at 37°C Celsius for 1 hour.

Colony PCR was performed for the prepared transformant, to select a transformed strain, and until removal of the selected transformant G recombinant vector was confirmed, by culturing at 37°C while performing cell streaking, the G recombinant vector was removed from the transformant. Specifically, by smearing in an ampicillin-added LB-agar plate and an LB-agar plate, respectively, a strain in which growth and development were confirmed in a kanamycin-added medium, but growth was not done in an ampicillin-added medium was selected.

In case of the colony PCR, considering that the efficiency of Taq polymerase is sharply reduced when amplifying DNA of 5kb or more, primers were designed so that about 1.0^{~}1.5kb regions were to be amplified to near 5' of the dhab1 gene inserted from the 100bp upstream region of the 5' homology arm region, to confirm whether the target gene was inserted, and the specific information of the primers was shown in Table 5 below.

**[Table 5]**

| Primer Name | Sequence (5'->3') | SEQ ID NO |
|---|---|---|
| poxB_For | agtgtgcgcaccagatgc | 32 |
| poxB_Rev | gtttgccgtccagttcgacga | 33 |
| nfrA_For | cgctctccgttacgttgattaatcg | 34 |
| nfrA_Rev | gtttgccgtccagttcgacga | 35 |
| mgsA_For | gaaggcagaaaacgctgtcg | 36 |
| mgsA_Rev | gtttgccgtccagttcgacga | 37 |
| IdhA_For | caatgatcggcggttcgc | 38 |
| IdhA_Rev | gtttgccgtccagttcgacga | 39 |
| yqhD_For | cacgtcgagtaaccgc | 40 |
| yqhD_Rev | gtttgccgtccagttcgacga | 41 |
| gldA_For | ctgcgggcgatcagcatatg | 42 |
| gldA_Rev | gtttgccgtccagttcgacga | 43 |
| glpK_For | gctgtttgcctgtttcgacaagc | 44 |
| glpK_Rev | gtttgccgtccagttcgacga | 45 |

### Comparative example 1. Preparation of strain in which 3-HP producing gene plasmid vector is inserted

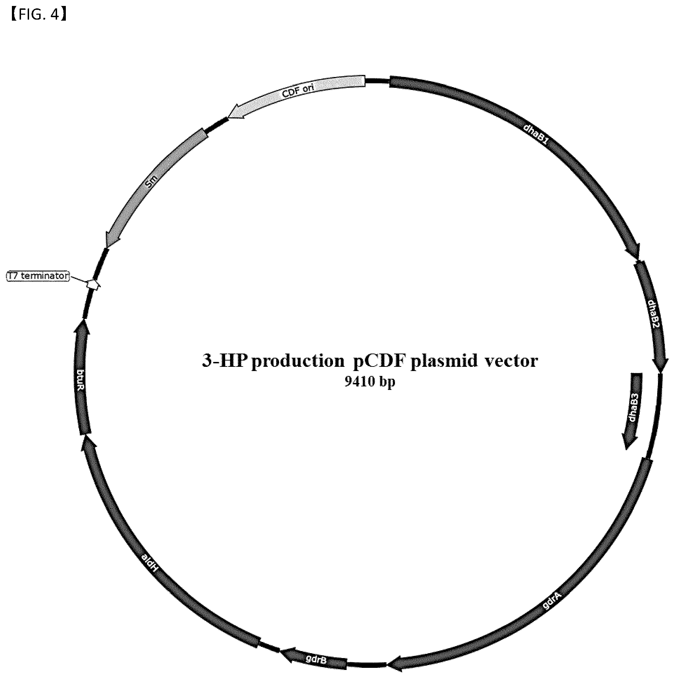

In order to proceed an experiment using a 3-HP producing gene plasmid vector, the pCDF_J23101_dhaB_gdrAB_J23100_aldH_btuR vector of Korean Patent Publication No. 10-2020-0051375 having 20^{~}30 copy numbers was prepared, and the structure of the corresponding vector was shown in FIG. 4.

In addition, by purifying with qiagen PCR purification kit, after treating xbal and bglll restriction enzymes (Thermo Scientific) to the pRSFDuet-1 (Merck Millipore 71341) vector, purified fragments of the vector were obtained. After that, for the pCDF_J23101_dhaB_gdrAB_J23100_aldH_btuR vector, PCR was performed using primers of Table 6 below, and by treating the xbal and bglll restriction enzymes and purifying with qiagen PCR purification kit, purified fragments of the 3-HP producing gene cassette recombinant vector were obtained.

**[Table 6]**

| Primer name | Primer sequence (5'->3') | SEQ ID NO: |
|---|---|---|
| 3-HP_For | ctagatttacagctagctcagtcc | 46 |
| 3-HP_Rev | tgccatattaataatcgatccctatctgc | 47 |

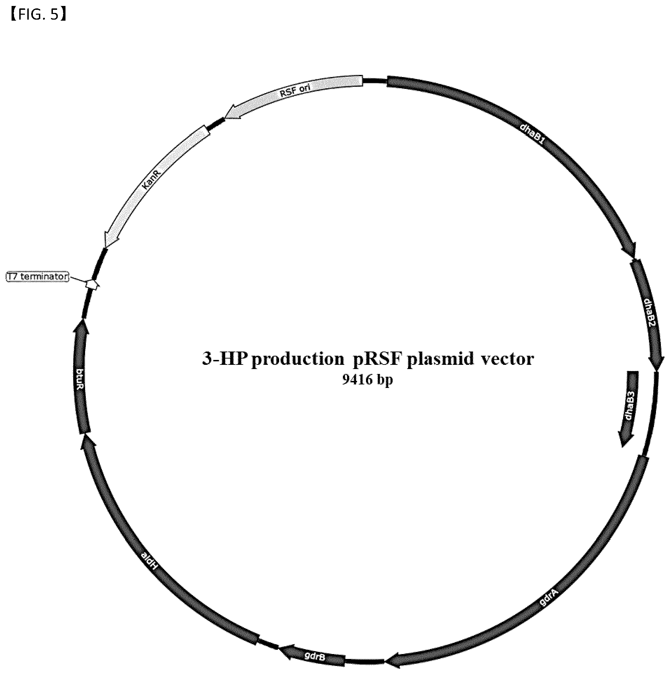

The purified fragments of the pRSFDuet-1 vector obtained through the purification process were connected with the purified fragments of the 3-HP producing gene cassette recombinant vector with NEB T4 ligase (NEB M0202S) to prepare a 3-HP producing gene plasmid vector, and the structure of the prepared vector was shown in FIG. 5.

The prepared pCDF_J23101_dhaB_gdrAB_J23100_aldH_btuR vector and pRSFDuet-1 vector-based 3-HP producing gene plasmid vector was transformed by introducing it into the DKALG strain of Example 2 by electroporation using an electroporation device (Eppendorf Eporator), thereby preparing a strain in which the 3-HP producing gene plasmid based on the pRSFDuet-1 vector was inserted (3-HP_pRSF) and a strain in which the pCDF_J23101_dhaB_gdrAB_J23100_aldH_btuR vector plasmid was inserted (3-HP_pCDF).

### Example 3. Cell 2-step culture and 3-HP production

Culture was carried out through a 2-step culture method using a minimal medium (M9_Gly) containing only glycerol as a carbon source for the strains in which the 3-HP producing gene prepared in Example 2 was inserted into genomic DNA with 5 copy numbers (3-HP_5gD), 6 copy numbers (3-HP_6gD) and 7 copy numbers (3-HP_7gD), respectively, and the strain in which the 3-HP producing gene plasmid vector prepared in Comparative example 1 was inserted with 20^{~}30 copy numbers (3-HP_pCDF) and the strain in which the 3-HP producing gene plasmid vector was inserted with 100 copy numbers or more (3-HP_pRSF).

The strains were inoculated with 1% in LB medium (growth medium) 50mL and grown at 37°C for 24 hours (first step culture), and whole cells were collected by centrifugation and the supernatant was discarded. Thereafter, the collected whole cells were added to a minimal medium containing purified water, M9 minimal medium salt, glycerol, magnesium sulfate and calcium chloride (M9_Gly; production medium) and cultured under the condition of 200rpm at 37°C for 24 hours or more (second step culture) to collect samples every predetermined time, and then confirm the 3-HP production by HPLC.

The LB medium is a medium prepared by dissolving BD Difco Miller Luria-Bertani product in DW (distilled water) at a concentration of 25g/L and then autoclave sterilizing at 121°C for 15 minutes, and the minimal medium (M9_Gly) is a medium containing Disodium phosphate 6g, Monopotassium phosphate 3g, Ammonium chloride 1g, Sodium chloride 0.5g, 1M CaCl₂ 0.1ml, 1M MgSO₄ 2ml, 5mM Vitamin B₁₂ 0.1ml and Glycerol 20g per 1L of medium. The HPLC measurement condition was shown in Table 7 below.

**[Table 7]**

| Detector | RI / UV detector |
|---|---|
| Column | Bio-Rad Aminex HPX-87H Ion Exclusion Column 300 mm x 7.8 mm |
| Mobile phase | 0.5 mM H2SO4 |
| Flow rate | 0.4 mL/min |
| Run time | 35 min |
| Column temperature | 35°C |
| Detector temperature | 35°C |
| Injection volume | 5 µL |

### Comparative example 2. Strain culture and 3-HP production in the conventional way

Additionally, for comparison with the conventional way, using the strain in which the 3-HP producing gene plasmid vector prepared in Comparative example 1 was inserted with 20^{~}30 copy numbers (3-HP_pCDF) and the strain in which the 3-HP producing gene plasmid vector was inserted with 100 copy numbers or more (3-HP_pRSF), they were cultured in a minimal medium containing Disodium phosphate 6g, Monopotassium phosphate 3g, Ammonium chloride 1g, Sodium chloride 0.5g, 1M CaCl2 0.1ml, 1M MgSO4 2ml, 5mM Vitamin B12 0.1ml, Glycerol 20g, Glucose 5g and 5% Streptomycin 0.1ml per 1L of medium (M9_Glu).

Specifically, the strains (3-HP_pCDF and 3-HP_pRSF) were inoculated in the LB medium of Example 3 and cultured under the condition of 37°C, 200RPM for 24 hours, and then the saturated culturing solution was inoculated in the minimal medium (M9_Glu) by 1 (v/v)% volume, and then, in the same manner, culturing which proceeds growth and production simultaneously was carried out under the condition of 37°C, 200RPM for 24 hours.

### Example 4. 3-HP production measurement result according to culture method

The cell concentration measured through absorbance measurement measured in Example 3 and Comparative example 2 above and the 3-HP concentration measured in Example 3 and Comparative example 2 above were shown in Table 8 below.

**[Table 8]**

| Type of used strain | Used production medium | Cell concentration (OD₆₀₀) | 3-HP concentration (g/L) | 3-HP concentration / cell concentration (OD₆₀₀) |
|---|---|---|---|---|
| 3-HP_5gD | M9_Gly | 3.84 | 4.06 | 1.057 |
| 3-HP_6gD | M9_Gly | 3.78 | 4.9 | 1.296 |
| 3-HP_7gD | M9_Gly | 3.63 | 5.3 | 1.460 |
| 3-HP_pCDF | M9_Glu | 0.429 | 0.1967 | 0.459 |
| 3-HP_pRSF | M9_Glu | 0.477 | 0.1311 | 0.275 |
| 3-HP_pCDF | M9_Gly | 2.395 | 4.8790 | 2.037 |
| 3-HP_pRSF | M9_Gly | 2.978 | 0.8319 | 0.279 |

As a result, in case of the strain in which the 3-HP producing gene was inserted into genomic DNA, a linear 3-HP concentration increase was confirmed as the gene copy number increased, and through this, it was confirmed that the corresponding strain was appropriate for confirmation of recombinant gene performance.

However, in case of the strain in which the plasmid vector was inserted, when comparing the 3-HP concentration produced by the 3-HP_pRSF strain with 100 copy numbers or more and 3-HP_pCDF with 20 ^{~} 30 copy numbers, it was not possible to confirm a 3-HP concentration increase according to the increase in the copy number, and through this, it was confirmed that the corresponding strain was not suitable for confirming recombinant gene performance.

Through this, it was confirmed that the target gene should be inserted into genomic DNA, not inserted in a plasmid vector form, in order to confirm recombinant gene performance.

### Example 5. Confirmation of change in 3-HP production according to increase in transduced copy number

In order to confirm the change in the 3-HP production of the strain transduced with a higher copy number (10, 12 copy numbers) than the strain in which the 3-HP producing gene transduction confirmed in Example 4 above was done with 5 and 7 copy numbers, the 3-HP production was confirmed in a 5L fermenter (Working volume 2L).

### 5-1. Recombinant vector and recombinant DNA preparation

By the substantially same method as Example 1-2 above, the G recombinant vector was prepared, and the G recombinant vector was prepared, using gRNA having nucleic acid sequences of Table 9 below (fhuA, fadL, adhE, pflB and aldA) additionally.

**[Table 9]**

| Target | gRNA sequence (5'->3') | Accession Number | SEQ ID NO | Name of the sequence |
|---|---|---|---|---|
| fhuA | TACGGCTGGTTGCCGAAAGAGGG | P06971 (UniProt) | 76 | fhuA_gRNA1 |
| | GCCAAAGATCCGGCAAACTCCGG | | 77 | fhuA_gRNA2 |
| fadL | GCACCGATTAACGACCAATTTGG | P0A8V6 (Uniprot) | 78 | fadL_gRNA1 |
| | CCTGAAATGTGGGAAGTGTCAGG | | 79 | fadL_gRNA2 |
| adhE | CGGGTGCGGGGAGAAGATAATGG | P0A9Q7 (Uniprot) | 80 | adhE_gRNA |
| pfIB | CATTACTTTGTCCCACAGGGTGG | P09373 (Uniprot) | 81 | pflB_gRNA |
| aldA | GATTCACGCGTCATTAATAGTGG | P25553 (Uniprot) | 82 | aldA_gRNA |

In addition, D recombinant DNA was prepared by the substantially same method as Example 1-3 above, and primers of Table 10 below were additionally used, and homology arms having sequences of Table 11 below were further used, to prepare D recombinant DNA.

**[Table 10]**

| homology arm primer | homology arm sequence (5'->3') | SEQ ID NO |
|---|---|---|
| fhuA_upstream_For | ACTGAACCGCTCTAGTACCAACAATAACGTAGATACCTGG | 83 |
| fhuA_upstream_Rev | TAGCTGTAAATCTAGAGACTGTCGCGCCGCAATA | 84 |
| fhuA_downstream_For | TACTAGCGCAGCTTAGCACCGTCTAAAGGTAAGCAGT | 85 |
| fhuA_downstream_Rev | CAGCAGCCTAGGTTATCCGACGGCGGCTGATGA | 86 |
| fadL_upstream_For | ACTGAACCGCTCTAGAAAACCTGACCCATAACACTC | 87 |
| fadL_upstream_Rev | TAGCTGTAAATCTAGGACCATAACCTCAATGATTTATTTT | 88 |
| fadL_downstream_For | TACTAGCGCAGCTTACGATTCACTATAGCCTGGC | 89 |
| fadL_downstream_Rev | CAGCAGCCTAGGTTAATTAACTTCATCATTGTTATTTTCA | 90 |
| adhE_upstream_For | ACTGAACCGCTCTAGAAAGCGCAGCGCCAGCTC | 91 |
| adhE_upstream_Rev | TAGCTGTAAATCTAGACAACGGCGTAATCTGTGCTTC | 92 |
| adhE_downstream_For | TACTAGCGCAGCTTACTTGCTCTTGAGTGAAACTGGCA | 93 |
| adhE_downstream_Rev | CAGCAGCCTAGGTTATAACGCGTACCCCAGACGATG | 94 |
| pflB_upstream_For | ACTGAACCGCTCTAGCAGTGATGTACTGTTTAGCCAGCCA | 95 |
| pflB_upstream_Rev | TAGCTGTAAATCTAGGTAAATCTGGTGTTCTGACCGGTCT | 96 |
| pflB_downstream_For | TACTAGCGCAGCTTAGTAACACCTACCTTCTTAAGTGGA | 97 |
| pflB_downstream_Rev | CAGCAGCCTAGGTTAGGCGAGATATGATCTATATCAATTT | 98 |
| aldA_upstream_For | ACTGAACCGCTCTAGATGCACCACGCTGGTTAAGT | 99 |
| aldA_upstream_Rev | TAGCTGTAAATCTAGTATTACCGGTCAAAAGAGCGGG | 100 |
| aldA_downstream_For | TACTAGCGCAGCTTAAAACCTTTGGCCCGGTGC | 101 |
| aldA_downstream_Rev | CAGCAGCCTAGGTTAGGTGCGAAGAAGGTGTTGATTTCC | 102 |

**[Table 11]**

| homology arm | homology arm sequence (5'->3') | SEQ ID NO |
|---|---|---|
| fhuA_upstream | taccaacaataacgtagatacctggtttgcgggcattgacggcagcacggtgaccatcacct gggtcggccgtgataacaaccagccgaccaaactgtatggtgccagcggggcaatgtcgat ttatcagcgttatctggctaaccagacgccaacgccgctgaatcttgttccgccagaagatat tgcagatatgggcgtggactacgacggcaactttgtttgcagcggtggcatgcgtatcttgcc ggtctggaccagcgatccgcaatcgctgtgccagcagagcgagatgcagcagcagccgtc aggcaatccgtttgatcagtcttctcagccgcagcaacagccgcaacagcaacctgctcagc aagagcagaaagacagcgacggtgtagccggttggatcaaggatatgtttggtagtaatta acatctaagcgtgaaataccggatggcgagttgccatccggtaaaataacatcccatctaa | 103 |
| | gatattaaccctttcttttcatctggttgtttattaacccttcaggaacgctcagattgcgtacc gcttgcgaacccgccagcgtttcgaatattatcttatctttataataatcattctcgtttacgtt atcattcactttacatcagagatataccaatggcgcgttccaaaactgctcagccaaaacac tcactgcgtaaaatcgcagttgtagtagccacagcggttagcggcatgtctgtttatgcacag gcagcggttgaaccgaaagaagacactatcaccgttaccgctgcacctgcgccgcaagaa agcgcatgggggcctgctgcaactattgcggcgcgacagtct | |
| fhuA_downstream | gcaccgtctaaaggtaagcagtatgaagtcggcgtgaaatatgtaccggaagatcgtccga ttgtagttactggtgccgtgtataatctcactaaaaccaacaacctgatggcggaccctgag ggttccttcttctcggttgaaggtggcgagatccgcgcacgtggcgtagaaatcgaagcgaa agcggcgctgtcggcgagtgttaacgtagtcggttcttatacttacaccgatgcggaataca ccaccgatactacctataaaggcaatacgcctgcacaggtgccaaaacacatggcttcgttg tgggctgactacaccttctttgacggtccgctttcaggtctgacgctgggcaccggtggtcgtt atactggctccagttatggtgatccggctaactcctttaaagtgggaagttatacggtcgtgg atgcgttagtacgttatgatctggcgcgagtcggcatggctggctccaacgtggcgctgcat gttaacaacctgttcgatcgtgaatacgtcgccagctgctttaacacttatggctgcttctggg gcgcagaacgtcaggtcgttgcaaccgcaaccttccgtttctaatttctcttttggggcacgg atttccgtgcccatttcacaagttggctgttatgcaggaatacacgaatcattccgataccac ttttgcactgcgtaatatctcctttcgtgtgcccgggcgcacgcttttgcatccgctgtcgttaa cctttcctgccgggaaagtgaccggtctgattggtcacaacggttctggtaaatccactctgc tcaaaatgcttggccgtcatcagccgccgtcgga | 104 |
| fadL_upstream | aaaacctgacccataacactcccctgtaaaaaaataaataaagtggtctgacctgatcata gtcttaaccatttttttacatttagccaagtggagaaaagggaaagtgggagctatgacaca gagagaaaagaagaagagaaaagaaaacgcccctgccagcaactgacaggggcgtacg tacccgagaggaattaacgcagacccagctggaaaatcagcatttcagcttcgcaggcaaa agtgaaatcgatatccaggcgcacaccgtcagactcttcagtgaaagtcggggtgattttgc aaggttcagattccacgctacgggctttttcagtcagcgccgccagcgtttgctctgcttctgc gcggtttgcaaacacgcggctgtaagacgcggtgcagtcggagttgtccataatggtgcca acatccatacagcagcaaaccggggtttcatcagcactacatttactcatcgttgatttcctct gtatgtgcacccaaggtgccagataaacgttgtggatattttacgcttccggaaagtgctgct ccagttgttaattctgcaaaatcggataagtgaccgaaatcacacttaaaaatgatctaaaa caaaattcacccgaatccatgagtgcgccacctccaaattttgccagctggatcgcgtttctt agatcatatttgaaaaaagatagaaacatacttgcaacattccagctggtccgacctatact ctcgccactggtctgatttctaagatgtacctcagaccctacacttcgcgctcctgttacagca cgtaacatagtttgtataaaaataaatcattgaggttatggtc | 105 |
| fadL_downstream | cgattcactatagcctggcttacaccagctggagtcagttccagcagctgaaagcgacctca accagtggcgacacgctgttccagaaacatgaaggctttaaagatgcttaccgcatcgcgtt gggtaccacttattactacgatgataactggaccttccgtaccggtatcgcctttgatgacag cccagttcctgcacagaatcgttctatctccattccggaccaggaccgtttctggctgagtgc aggtacgacttacgcatttaataaagatgcttcagtcgacgttggtgtttcttatatgcacggt cagagcgtgaaaattaacgaaggcccataccagttcgagtctgaaggtaaagcctggctgt tcggtactaactttaactacgcgttctgataacgcgttcgcctggataaagtcacctgcatag caggtgactttaactcccccacttcaccgaaagtagtgtcctcattgcttacctcctgagtttt gcaaacagcctgttggcagcttgccctttcaaatcaataagcggtatccacagacaaacctg aagggaaaggcatattttcaggcgttctgctcgtccttctcaaagagtttacttttctgcatttc caggatactccccccccctggctattgtgcgctcatacactcaaattaaagataggttctaaa taaatgagcgttttttgatagtctatttcattaggtaatatatatttgtaacaaatcaatcaaa atggaataaaatcatgctaccatctatttcaatcaacaataccagcgcagcttacccagaat ccatcaatgaaaataacaatgatgaagttaat | 106 |
| adhE_upstream | aaagcgcagcgccagctcttcgaagtgagtttccggatgttcgtacataacccacatgatctt | 107 |
| | cgcggcgtccatcggggaaccaccacccagcgcgataatcacgtctggtttgaaggagtttg ccagttctgcacctttacgaacgatgctcagggtcgggtccgcttctacttcgaagaagactt cagtttcaacgcctgctgctttcagtacggaagtgatctgatcagcataaccattgttgaaca ggaagcggtcagtcacgatgagcgcacgtttgtggccatcagtaatcacttcatccagcgcg attggcagggagccacggcggaagtagatagatttcggaagtttgtgccacaacatgttttc agctcgcttagcaacggttttcttgttgatcaggtgtttcggaccaacgttttcagagatggag ttaccaccccaagaaccacaacccagagtcagggaaggtgcgagtttgaagttatacaggt caccgataccaccctgagacgctggggtgttaatcaggatacgcgccgttttcattttctgac cgaagtaagaaacgcgagccggttggttatcctggtcagtgtacaggcaagaggtatgacc gataccgcccatagcaaccagtttctctgctttttctaccgcgtcttcgaaatctttagcgcgg tacattgccagagtcggggacagtttttcatgtgcgaacggttcgctttcatcaacaacggtc acttcaccgatcagaatcttggtgttttctggtacagagaagcctgccagttcagcaattttat aggctggctgaccaacgatagccgcgttcagcgcaccgtttttcaggataacatcctgaaca gctttcagctctttaccctgcaacagatagccgccgtgggttgcaaaacgttcacgtacagc gtcataaacagagtcaacaacaacaacagactgttcagaagcacagattacgccgttgt | |
| adhE_downstream | cttgctcttgagtgaaactggcatattcacgctgggctttttttacacgctctacgagtgcgtt aagttcagcgacattagtaacagccataatgctctcctgataatgttaaacttttttagtaaat catctgctcgaatacgagagtatagtcagtgcggtgatgatttgcttaacctatgaaaatca aaagcttactcgcgctcacactcactgtgatttactaaaagagtttaaacattagagttatta tctctaatgcgtcacttccaggtggcgtaagcaagattactcacttctgggtactgattacgtg atccaaatcaaatttttgcaaagctgacacctttcagcatcgcttttcgccattatagctaaca gttaataaattgtagtatgatttggtggctacattagcatgttttgcacaactagataacaat aacgaatgatagcaattttaagtagttaggaggtgaaaaatgctgtcaaaaggcgtattgtc agcgcgtcttttcaaccttatttatggctaacattatccggcttttgcttcggagctaaccgtg attcagaccttttttgattttcccgtttacttcaaatttttcatcgggttatttgcgctggtcaac ccggtagggattattcccgtctttatcagcatgaccagttatcagacagcggcagcgcgaaa caaaactaaccttacagccaacctgtctgtggccattatcttgtggatctcgctttttctcggc gacacgattctacaactttttggtatatcaattgattcgttccgtatcgccgggggtatcctgg tggtgacaatagcgatgtcgatgatcagcggcaagcttggcgaggataaacagaacaagc aagaaaaatcagaaaccgcggtacgtgaaagcattggtgtggtgccactggcgttgccgtt gatggcggggccaggggcgatcagttctaccatcgtctggggtacgcgtta | 108 |
| pflB_upstream | cagtgatgtactgtttagccagccagtccatgaagtgatccatgcgctccatcacttcatcat agttcaggacatcgcctttgatcggttcagacttcggaccaacctgcattttcagtttttcgtc aacgccgccgttgattgcgtacagcatggttttcgccaggtttgcacgcgcaccgaagaact gcatttgtttaccaacgatcatcgggcttacgcagcaagcaatagcgtagtcatcgttgttga agtccggacgcatcaggtcatcgttctcatactgcagagaagaggtgtcgatggacacttta gcggcgaatttcttgaagttcagcggcagtttttcagaccacagaatggtcatgttcggttcc ggagacggacccatggtgtacagggtgttcaggaaacggaagctgtttttggtaaccaggg tacgaccgtcgaggcccataccaccgatagattcggttgcccagatcgggtcgccagagaa cagttcatcgtattccggagtacgcaggaagcgaaccatacgcagtttcatgaccaggtggt caaccatttcctgcgcttcttgttcggtgatcttgccagctttcaggtcacgttcgatgtacaca tccaggaaggtggaggtacgaccgaaggacattgcagcaccgttctgagacttaacagca gccaggtagccgaagtaagtccactggatagcttcctgagcgttggtagccggaccagaga tgtcgtagccgtatttcgcagccatttctttcatctgacccagagcgcggtgctgttcagcgat ttcttcgcgcagacggatagtctgttccaggtttacgccgttttccagatcagcctgcagaga agtgaactgtgccagtttgtctttcatcaggtagtcgataccgtacagcgcaacgcgacggt agtcaccgatgatacggccacggccatatgcatctggcagaccggtcagaacaccagattt ac | 109 |
| pflB_downstream | gtaacacctaccttcttaagtggattttttatttactgcgtacttcgacaaccattaatggtggt | 110 |
| | cgttttcacgcaggtaaatgacccagtatgtcaacccaaccaacaaaccaccaccgataat gttgccgatcgtaaccggaatcaggttatcagtgatgaaattcatcacggtcaggtgagaaa aattttccggtgcagaaccgactgcggtccaaaattccggggatgcgaagtcgcggattac aatacccatcgggatcataaacatgtttgcgatactgtgctcaaaaccgctggcaacaaac atcgcgaccggcagcaccataatgaacgctttgtccatcaggctgcggccagaataactcat ccatactgccagacataccatcaggtttgccaggataccaagacagacggcctcaataaaa gtatggtgcactttgtggtcggcggtttgtaggacgtttagtccccattgaccatttgcggtca tatactcgccggaaagccacattaaaagtacaaacagcagtgcgccgaccaggttgccaa aatagacatttagccagtttttcgccaactgaccccaggtgatgcgcccactcgccttagcaa caacaatcaacacggtggaagtaaagagatcggctccgcagacaacacaaagaatcagc cccagagagaagcaaatgccgccaaccagttttgccatgccgaagggcattgtgcctgtgc cagtggttgctgtgatatagaagacgaatgcgattgagatgaaaacaccggcggtaatcgc cagatagaaagtcttaagcggatgtttcgttgctttatagacacccgcctcttcggccactttg gccattgcagcaggaagtaaaagatcaaaagggttgtcagctttcacactaactctctcttt attaagtcggcgacgagatactaacaaagcattatagatgagaaattgatatagatcatat ctcgcc | |
| aldA_upstream | atgcaccacgctggttaagttatcccggattcgttcctcagttaggaaataacgcagacagt gtaatctttattaatcagttacaaggattatggccagttgagcgttatctctcactactcactg gcgagctgccgcgtttacgcgatgatagcgatggctacggtccccgcgggcgagattttatc gttcacgttgattttccggcagaagtcatccatgcatggcaaacgctgaaacatgatgcggt gctcatcgaggcgatggaaagtcgctcgttacgttaaaaattgcccgtttgtgaaccacttgt ttgcaaacgggcatgactcctgacttttatttctgccttttattccttttacacttgtttttatgaa gcccttcacagaattgtcctttcacgattccgtctctctgatgattgatgttaattaacaatgta ttcaccgaaaacaaacatataaatcacaggagtcgcccatgtcagtacccgttcaacatcct atgtatatcgatggacagtttgttacctggcgtggagacgcatggattgatgtggtaaaccct gctacagaggctgtcatttcccgcatacccgatggtcaggccgaggatgcccgtaaggcaat cgatgcagcagaacgtgcacaaccagaatgggaagcgttgcctgctattgaacgcgccagt tggttgcgcaaaatctccgccgggatccgcgaacgcgccagtgaaatcagtgcgctgattgt tgaagaagggggcaagatccagcagctggctgaagtcgaagtggcttttactgccgactat atcgattacatggcggagtgggcacggcgttacgagggcgagattattcaaagcgatcgtc caggagaaaatattcttttgtttaaacgtgcgcttggtgtgactaccggcattctgccgtgga acttcccgttcttcctcattgcccgcaaaatggctcccgctcttttgaccggtaata | 111 |
| aldA_downstream | aaacctttggcccggtgctgccagttgtcgcatttgacacgctggaagatgctatctcaatgg ctaatgacagtgattacggcctgacctcatcaatctatacccaaaatctgaacgtcgcgatg aaagccattaaagggctgaagtttggtgaaacttacatcaaccgtgaaaacttcgaagcta tgcaaggcttccacgccggatggcgtaaatccggtattggcggcgcagatggtaaacatgg cttgcatgaatatctgcagacccaggtggtttatttacagtcttaatgagtgaaagaggcgg aggttttttcctccgcctgtgcgcgtcagagtttagcgaatttttcgagggtgcgaataagctg tgtgacgaagccatattcgttatcgtaccaggcgaccgttttcaccagttgtaaatcgcccac ggcggtaatttccgtttgcgtggcatcaaacaccgaaccgaaatggctgccaatgatatcgg aagagactatttcttcatcggtataaccaaatgactcgttattggtggttgcttgtttaagtgc gttattcacctcttcggcagtcacttttttccgagaatcgataccagttcagtgaccgaacctg ttttcaccggcacgcgttgcgcatgacctttcagtttgccgctcagttccgggatcaccagac caatggcttttgccgcccccgtagtgtggggaatgatattttctgccgctgcgcgtgaagcac gtaaatctttaccacgcgggccatccaccagtgactgggtgccagtataggcatgaatggtc gtcatcgtgccgacttctatcccgaaactgtcatgcaaggctttggccatcggcgcaagaca gttagtggtgcatgacgccacggaaacaatggtgtcgttgccatccagagtgtcgtcattga cgttataaacgatagttttcatttcaccggcaggggcggaaatcaacaccttcttcgcacc | 112 |

### 5-2. Gene deletion and preparation of strain in which 3-HP producing gene is inserted into genomic DNA

The common recombinant vector prepared in Example 1-1, G recombinant vector and D recombinant DNA prepared in Example 5-1 were transduced into W3110 which was a strain in which yqhD, glpK, IdhA, ack-pta and gldA genes were deleted from *E. coli* W3110 strain (purchased from KCTC) (ΔyqhD, ΔgIpK, ΔIdhA, Δack-pta and LgIdA) (named as DKALG strain) with 5 copy numbers, 7 copy numbers, 10 copy numbers and 12 copy numbers, respectively. After that, a transformant was prepared by expressing Cas9 and RED recombinase of the common recombinant vector.

The 5-copy number and 7-copy number transducing was performed by the substantially same method as Example 2 above, and the 10-copy number transducing was transducing using IdhA, gldA, glpK, yqhD, mgsA, poxB, nfrA, fhuA, fadL and adhE (10) sgRNA of Example 5-1 above, and the 12-copy number transducing was transducing using IdhA, gldA, glpK, yqhD, mgsA, poxB, nfrA, fhuA, fadL, adhE, pflB and aldA (12) sgRNA. In addition, in order to confirm insertion of the target gene, using primers of Table 12 below in addition to the primers used in Example 2 above, insertion of the target gene was confirmed.

**[Table 12]**

| Primer Name | Primer Sequence (5'->3') | SEQ ID NO |
|---|---|---|
| fhuA_For | tggaccatgcagcaggtggtacaac | 113 |
| fhuA_Rev | gtttgccgtccagttcgacga | 114 |
| fadL_For | gtacgtaaaccgctaacaatggcga | 115 |
| fadL_Rev | gtttgccgtccagttcgacga | 116 |
| adhE_For | ttcgggaacttgtagatacgtttac | 117 |
| adhE_Rev | gtttgccgtccagttcgacga | 118 |
| pflB_For | gtacttgtcgtgcatgtagtgga | 119 |
| pflB_Rev | gtttgccgtccagttcgacga | 120 |
| aldA_For | gttccgccgtatgactgg | 121 |
| aldA_Rev | gtttgccgtccagttcgacga | 122 |

### 5.3 Cell 2-step culture and 3-HP production

For the strains in which the 3-HP producing gene prepared in Example 5.2 was inserted into genomic DNA with 5 copy numbers (3-HP_5gD), 7 copy numbers (3-HP_7gD), 10 copy numbers (3-HP_10gD) or 12 copy numbers (3-HP_12gD), respectively, high concentration cell culture was performed in a 5L fermenter (Working volume 2L) by a fed-batch culture method.

Specifically, glucose 20g/L, and an antibiotic for selection (streptomycin) 25mg/L was added to MR medium (KH₂PO₄ 6.67g, (NH₄)₂HPO₄ 4g, MgSO₄-7H2O 0.8g, citric acid 0.8g, and trace metal solution 5mL per 1L; herein, Trace metal solution was 5M HCl SmL, FeSO₄-7H₂O 10g, CaCl₂ 2g, ZnSO₄·7H₂O 2.2g, MnSO₄·4H₂O 0.5g, CuSO₄·5H₂O 1g, (NH₄)₆Mo₇O₂·4H₂O 0.1g, and Na₂B₄O₂·10H₂O 0.02g per 1L) and it was used as a cell culture medium, and the temperature of 35°C Celsius was maintained. pH was maintained at 6.95 using ammonia water, and the dissolved oxygen amount (DO) was maintained at 20% while increasing the stirring speed stepwise up to 900rpm, and the aeration was maintained at 1vvm, and the fed-batch culture was performed using the pH-stat feeding method, and glucose was added at 3g/L.

After completing the high concentration cell culture of 20 hours, the cell culture solution was centrifuged at 6,000rpm at 4 degrees Celsius for 10 minutes to recover cells. The recovered cells were suspended in PBS (phosphate-buffered saline) and used in the next step.

The medium for 3-HP production was prepared by adding 70g/L of glycerol and 50uM of vitamin B₁₂ to M9 medium comprising no glucose. The cell suspension prepared above was inoculated into the medium for 3-HP production so that the cell inoculum amount was 5g/L (based on dry cell weight), and the 3-HP production step was performed in a 5L fermenter (Working volume 2L). The culture condition for 3-HP production was that the temperature was maintained at 35°C Celsius, and the stirring speed was maintained at 300rpm, and the aeration was maintained at 1 vvm, and the pH was maintained at 7.0 using Ca(OH)₂, and it was proceeded for 24 hours.

The 3-HP concentration over time in the 3-HP production process was measured by high pressure liquid chromatography (High Pressure; HPLC), and the result was shown in Table 13 below.

**[Table 13]**

| Type of used strain | 3-HP concentration (g/L) |
|---|---|
| 3-HP_5gD | 64.6 |
| 3-HP_7gD | 75.6 |
| 3-HP_10gD | 91.0 |
| 3-HP_12gD | 115.5 |

As a result, as confirmed in Example 4 above, in case of the strain in which the 3-HP producing gene was inserted into genomic DNA, a linear 3-HP concentration increase was confirmed as the gene copy number increased, and in particular, in case of inserting the gene of 10 copy numbers and 12 copy numbers, a linear 3-HP concentration increase was confirmed. Through this, it was confirmed that the corresponding strain was suitable for confirmation of recombinant gene performance, and inserting a target gene into genomic DNA for confirmation of recombinant gene performance was appropriate.

## Claims

1. An *Escherichia coli* bacterium having 3-hydroxypropionic acid (3-hydroxypropionate, 3-HP) producing ability, in which a foreign 3-HP producing gene is inserted into genomic DNA with at least one copy number.

2. The *E. coli* bacterium having 3-HP producing ability according to claim 1, wherein the 3HP producing gene comprises a gene of at least one enzyme selected from the group consisting of glycerol dehydratase, aldehyde dehydrogenase, glycerol dehydratase reactivase and adenosyltransferase.

3. The *E. coli* bacterium having 3-HP producing ability according to claim 1, wherein at least one gene selected from the group consisting of yqhD, glpK, IdhA, ack-pta and gldA is deleted among genes of the *E. coli.*

4. The *E. coli* bacterium having 3-HP producing ability according to claim 1, wherein the 3-HP producing gene is inserted where at least one gene selected from the group consisting of IdhA, gldA, glpK, yqhD, mgsA, poxB, nfrA, fhuA, fadL, adhE, pflB and aldA genes of the *E. coli* is located.

5. The *E. coli* bacterium having 3-HP producing ability according to claim 1, wherein the 3-HP producing gene is inserted into genomic DNA by CRISPR-Cas9 system.

6. The *E. coli* bacterium having 3-HP producing ability according to any one claim of claim 1 to claim 5, wherein the 3-HP producing ability is increased in proportion to the copy number of the 3-HP producing gene.

7. A composition for determining 3-HP producing ability of a 3-HP producing gene, wherein the composition comprises the *E. coli* bacterium of any one of claims 1 to 5, and the 3-HP producing gene is inserted into genomic DNA of the *E. coli* bacterium.

8. The composition according to claim 7, wherein the determining 3-HP producing ability is confirming whether the 3-HP production by the *E. coli* bacterium is proportional to the copy number of the 3-HP producing gene inserted into genomic DNA of the *E. coli* bacterium.
